(19) Europäisches Patentamt
European Patent Office
Office européen des brevets

(11) EP 2 953 611 B1

(12) **FASCICULE DE BREVET EUROPEEN**

(45) Date de publication et mention de la délivrance du brevet:
**18.08.2021 Bulletin 2021/33**

(21) Numéro de dépôt: **14704136.2**

(22) Date de dépôt: **11.02.2014**

(51) Int Cl.:
*A61K 8/60* (2006.01) *A61K 8/9789* (2017.01)
*A61Q 5/02* (2006.01) *A61Q 17/04* (2006.01)
*A61Q 19/00* (2006.01) *A61Q 19/08* (2006.01)
*A61Q 19/10* (2006.01) *A61K 45/06* (2006.01)
*A61K 31/22* (2006.01) *A61K 31/23* (2006.01)
*A61K 31/232* (2006.01) *A61K 31/7012* (2006.01)
*A61K 31/7024* (2006.01) *A61K 36/54* (2006.01)
*A61P 17/02* (2006.01) *A61P 35/00* (2006.01)

(86) Numéro de dépôt international:
**PCT/EP2014/052665**

(87) Numéro de publication internationale:
**WO 2014/122326 (14.08.2014 Gazette 2014/33)**

(54) **UTILISATION D'UNE COMPOSITION COMPRENANT UN PERSÉOSE D'AVOCAT DANS LA PROTECTION DES CELLULES SOUCHES ÉPIDERMIQUES**

VERWENDUNG EINER ZUSAMMENSETZUNG MIT AVOCADO-PERSEOSE ZUM SCHUTZ VON EPIDERMALEN STAMMZELLEN

USE OF A COMPOSITION COMPRISING AVOCADO PERSEOSE IN THE PROTECTION OF EPIDERMAL STEM CELLS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.02.2013 FR 1351136**
**11.02.2013 US 201361763294 P**

(43) Date de publication de la demande:
**16.12.2015 Bulletin 2015/51**

(73) Titulaire: **Laboratoires Expanscience**
**92048 Paris La Défense Cedex (FR)**

(72) Inventeurs:
- **MSIKA, Philippe**
  **F-78000 Versailles (FR)**
- **BAUDOUIN, Caroline**
  **F-78120 Rambouillet (FR)**

(74) Mandataire: **Regimbeau**
**20, rue de Chazelles**
**75847 Paris Cedex 17 (FR)**

(56) Documents cités:
**WO-A1-2005/115421 WO-A1-2011/073281**
**WO-A1-2012/085224 WO-A1-2012/123774**
**JP-A- 2003 226 655**

- **S Leclere-Bienfait ET AL: "Avocado perseose, a biomimetic active ingredient for the protection and accompaniment of infants' skin", Journal of investigative dermatology, 1 mai 2013 (2013-05-01), page S106, XP055095186, DOI: http://www.nature.com/jid/journal/v133/n1s/index.html#ab Extrait de l'Internet: URL:http://www.nature.com/jid/journal/v133/n1s/pdf/jid201399a.pdf [extrait le 2014-01-08]**
- **BREDIF S ET AL: "AVOCADO SUGARS ARE EFFECTIVE INDUCERS OF CUTANEOUS DEFENSIVE FUNCTIONS", THE JOURNAL OF INVESTIGATIVE DERMATOLOGY : OFFICIAL JOURNAL OF THE SOCIETY FOR INVESTIGATIVE DERMATOLOGY AND THE EUROPEAN SOCIETY FOR DERMATOLOGICAL RESEARCH, ELSEVIER, US, vol. 126, no. SUPPL 3, 7 September 2006 (2006-09-07), page 52, XP009081682, ISSN: 0022-202X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 953 611 B1

Printed by Jouve, 75001 PARIS (FR)

**(Cont. page suivante)**

- **Board: "High K<sub>m</sub> glucose-phosphorylating (glucokinase) activities in a range of tumor cell lines and inhibition of rates of tumor growth by the specific enzyme inhibitor mannoheptulose", Cancer research, vol. 55, no. 15, 1 January 1995 (1995-01-01), pages 3278-3285, XP055096047, ISSN: 0008-5472**


Remarques:

Le dossier contient des informations techniques présentées postérieurement au dépôt de la demande et ne figurant pas dans le présent fascicule.

## Description

### Introduction

Avec une surface d'environ 2 $m^2$, la peau forme l'organe le plus important du corps humain (environ 16 % du poids corporel). Elle a pour fonction principale d'établir une barrière de protection contre les atteintes de l'environnement tout en permettant certains échanges entre le milieu intérieur et le milieu extérieur. Elle est le siège de nombreux processus métaboliques qui sont modulés par les conditions physiologiques de l'organisme et les conditions de l'environnement. La peau est formée de deux couches jointes : l'épiderme et le derme auxquelles on peut associer les tissus sous-cutanés.

L'épiderme constitue la structure la plus superficielle de la peau. Son principal rôle est la protection du corps : il assure l'imperméabilité de la peau et sa résistance. Ce tissu est constitué en particulier d'un épithélium pluristratifié, appelé épithélium interfolliculaire, dont le rôle est notamment d'assurer une fonction de barrière vis-à-vis de l'environnement. On peut identifier dans celui-ci 4 couches cellulaires distinctes, une couche basale (*stratum basalis*), une couche épineuse (*stratum spinosum*), une couche granuleuse (*stratum granulosum*), et une couche cornée (*stratum corneum*).

Si différents types cellulaires coexistent dans l'épiderme, les kératinocytes sont largement majoritaires (90 %). Leur activité caractéristique est la synthèse des kératines qui représentent 95 % des protéines totales de l'épiderme.

Toutefois, le *stratum corneum,* qui est la couche protégeant la peau contre les agressions extérieures (chaleur, froid, déshydratation, etc.), est composé de cellules spécifiques, les cornéocytes, qui sont des cellules complètement aplaties, sans noyau ni organites cytoplasmiques, et sont le résultat de l'ultime phase de différenciation des kératinocytes. Le *stratum corneum* est plus particulièrement divisée en deux couches distinctes, l'une où les cornéocytes sont encore reliés les uns aux autres grâce aux cornéodesmosomes, et une autre où, sous l'action d'enzymes spécifiques, les cornéodesmosomes se dégradent, permettant aux cornéocytes de se détacher : c'est la desquamation. Ce processus participe au renouvellement continu de l'épiderme.

L'adaptation à la vie extra-utérine est un processus qui commence dès la naissance et se poursuit tout au long de la première année de la vie. Les premiers mois de la vie postnatale constituent une période de réorganisation structurelle et fonctionnelle de la peau qui permet une adaptation physiologique à l'environnement extra-utérin. Par exemple, l'immaturité de la peau du nouveau-né est soulignée par la différence de structure et de composition moléculaire du stratum corneum par rapport à l'adulte. Celles-ci sont incomplètes et continuent ainsi de se développer durant les 12 premiers mois au moins après la naissance (Chiou et al., Skin Pharmacol Physiol, 17: 57-66, 2004; Nikolovski et al., J Invest Dermatol, 128: 1728-1736, 2008; Stamatas et al., Pediatr Dermatol, 27: 125-131, 2010).

Par ailleurs, les résultats d'une étude clinique récente (Fluhr et al., Br J Dermatol, 166(3) : 483-90, 2012) suggèrent que la peau des nourrissons présente une certaine immaturité sur sa capacité à capter l'eau et à réguler les mécanismes qui y sont liés, ce qui aurait un impact sur la qualité et la compétence de la fonction barrière.

Une maturation incomplète de la peau peut avoir des conséquences cliniques importantes. Il est donc important de permettre à la peau de se construire et de se développer correctement et harmonieusement, sans quoi son organisation fonctionnelle et structurelle pourrait en être obérée. À cet égard, il est crucial de préserver la capacité de renouvellement de l'épiderme.

L'épiderme interfolliculaire est un tissu dynamique qui se renouvelle constamment, dans un délai d'environ 28 jours en moyenne. Le *stratum corneum,* quant à lui, est complètement renouvelé tous les 15 jours Le maintien de l'intégrité de l'épiderme est intimement lié à la biologie des kératinocytes constituant la couche basale de celui-ci. L'homéostasie épidermique résulte en effet d'un équilibre finement régulé entre prolifération et différenciation des kératinocytes basaux, qui permet d'assurer le renouvellement physiologique du tissu, ainsi que sa régénération en situation de lésion. Plus particulièrement, la capacité régénérative de l'épiderme est conférée par des cellules souches adultes qui permettent le remplacement régulier des cellules différenciées éliminées lors de la kératinisation. Ce processus est en particulier crucial pour la maturation et le maintien de la fonction barrière.

Les cellules souches adultes sont des cellules non différenciées mais spécialisées qui peuvent être définies par deux propriétés principales : leur aptitude à s'auto-renouveler et à se maintenir en place durant de très longues périodes, mais également leur capacité à générer tous les types de cellules différenciées du tissu en question, ce qui définit leur multipotence. Toutefois, cette dernière propriété peut être absente chez certaines cellules souches adultes ; en revanche, tous les types de cellules souches adultes présentent un potentiel élevé de prolifération à long terme. A ces deux caractéristiques principales s'ajoute une certaine quiescence de ces cellules. Ces cellules se divisent peu fréquemment et uniquement sous l'influence de stimuli précis et probablement spécifiques du tissu dans lequel elles sont situées. La division, dite asymétrique, va donner deux cellules filles aux destins distincts. La première sera une cellule souche identique à la cellule mère, qui sera dotée des mêmes caractéristiques (auto-renouvèlement et multipotence) et qui va entrer en quiescence. La seconde va en revanche entrer dans le cycle cellulaire et se diviser activement un certain nombre de fois en perdant son caractère multipotent, avant de s'engager dans une voie de différenciation définitive.

La peau, en tant qu'organe au renouvellement rapide et continu, fait ainsi partie des tissus qui hébergent un nombre significatif de cellules souches ou déjà engagées dans un lignage spécifique. Ainsi dans la peau, on trouve, entre autres, des cellules souches mélanocytaires (qui donnent des mélanocytes) se trouvant dans les follicules pileux et dans la couche basale de l'épiderme, les cellules dites SKP positives pour la nestine au niveau neural, les cellules souches dermiques/mésenchymateuses dans le tissu conjonctif, les cellules souches dérivées du tissu adipeux et qui sont dans le tissu adipeux et les cellules souches épidermiques.

Les cellules souches épidermiques résident dans la région du bulge/renflement du follicule pileux (cellules souches folliculaires), dans les glandes sébacées (cellules souches sébacées) et dans la couche basale de l'épiderme (cellules souches épidermiques interfolliculaires) (Dahl, J Cosmet Dermatol, 11: 297-306, 2012 ; Eckert et al, Biochim Biophys Acta, 1830 : 2427-2434, 2013). Les cellules souches épidermiques ne forment pas seulement les kératinocytes générant l'épiderme et sa fonction barrière (stratum corneum) mais elles forment aussi les follicules du cheveu et les glandes sébacées et autres annexes.

Le réservoir majeur de cellules souches épidermiques est la couche basale, laquelle contient des cellules souches qui assurent le renouvellement des kératinocytes de l'épiderme. En effet, ces cellules souches donnent naissance par division asymétrique à des cellules amplificatrices transitoires (transit amplifying cells), lesquelles sont vouées à une différenciation définitive après s'être divisées 3 à 5 fois. L'étape suivante de différenciation des cellules amplificatrices transitoires génère les kératinocytes basaux post-mitotiques (PMD) qui ne se divisent plus et ont entamé le processus de différenciation et de migration en s'éloignant de la membrane basale (Fuchs, J Cell Biol, 182(2) : 273-284, 2008). Ces processus sont finement contrôlés et régulés en relation avec les processus de prolifération, différenciation et desquamation.

Les cellules souches résident dans un microenvironnement spécialisé qui aide au maintien du phénotype souche des cellules souches. Ce microenvironnement appelé « niche » crée un contexte biochimique, structural et spatial qui influence la division et la différenciation cellulaire. Chaque niche est généralement constituée de cellules souches, de cellules amplificatrices transitoires, de macromolécules de la matrice extracellulaire, de molécules de signalisation et autres facteurs (Dahl, J Cosmet Dermatol, 11: 297-306, 2012).

Les cellules souches épidermiques participent au maintien de l'homéostasie de la peau. Elles doivent assurer l'intégrité du tissu tout au long de la vie en régénérant les couches de cellules cutanées soumises au programme de différenciation. Elles sont aussi essentielles pour les phénomènes de cicatrisation et de réparation de l'épiderme en cas de blessure. Mais, une perte de contrôle de l'asymétrie des divisions peut appauvrir le pool de cellules souches, ce qui entraine des défauts de cicatrisation et des changements dégénératifs associés au vieillissement (Dahl, J Cosmet Dermatol, 11: 297-306, 2012). De plus avec le temps, les cellules souches perdent leur potentiel souche et la peau vieillit (Dereure, Ann Dermatol Venereol. 139(8-9) : 568-578, 2012). Le vieillissement de ces cellules est accéléré par des facteurs extrinsèques comme la pollution et les rayons UV. La protection des cellules souches est donc essentielle pour limiter les agressions extérieures. De façon plus générale, il est donc crucial pour la bonne santé de la peau de conserver intact le potentiel des cellules souches et, en particulier, leur capacité d'auto-renouvèlement.

## Description

Les inventeurs ont trouvé de manière surprenante que des sucres en C7 et dérivés de formule (I), appelés perséose d'avocat et que l'on définira plus loin, présentent, entre autres, une activité protectrice des cellules souches épidermiques. Ces sucres en C7 et dérivés sont capables d'une façon surprenante de maintenir l'expression des marqueurs de cellules souches adultes, notamment les cellules souches basales. Ainsi, ces composés ont un effet bénéfique sur la conservation du potentiel des cellules souches épidermiques et, ainsi, contribuent à maintenir l'homéostasie de la peau.

L'invention est notamment telle que définie dans les revendications. L'invention a notamment pour objet l'utilisation cosmétique d'une composition comprenant au moins un sucre en C7 ou dérivé de formule (I) suivante

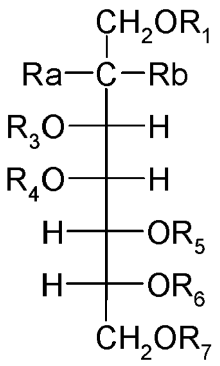

(I)

dans laquelle
Ra représente un atome d'hydrogène et Rb représente un -$OR_2$ ou CRaRb représente le radical CO ;
$R_1$, $R_2$, $R_3$, $R_4$, $R_5$, $R_6$ et $R_7$ représentent, indépendamment l'un de l'autre

- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-$OC_2H_5$) et groupement -$SO_3M$ avec M représentant un atome d'hydrogène, un ion ammonium $NH_4^+$ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-$OC_2H_5$) et groupement -$SO_3M$ avec M représentant un atome d'hydrogène, un ion ammonium $NH_4^+$ ou un ion métallique ;

et un excipient pharmaceutiquement acceptable
pour la prévention du vieillissement de la peau, caractérisée en ce que ladite composition protège les cellules souches épidermiques, notamment les cellules souches basales, et en ce qu'elle est destinée aux enfants.

Il est ici décrit une utilisation du perséose d'avocat pour protéger les cellules souches épidermiques et, en particulier, les cellules souches basales.

En particulier, selon un aspect qui ne fait pas partie de l'invention telle que revendiquée, le perséose d'avocat permet de prévenir les effets délétères des agressions environnementales.

Par « agression environnementale », on entend désigner ici toutes les conditions de l'environnement qui exercent une contrainte sévère sur la peau et affectent le processus naturel de construction, d'évolution et de maturation de celle-ci.

De telles conditions comprennent par exemple le chauffage et la climatisation, le vent, l'exposition au soleil, et l'irradiation aux UV en résultant, et la pollution de l'environnement. A ce sujet, il faut noter que certaines conditions peuvent affecter le processus naturel de construction, d'évolution et de maturation de la peau en accélérant ledit processus. Mais il est aussi possible que certaines conditions expérimentales limitent ce processus (voir par exemple Valacchi et al., Ann. N.Y. Acad. Sci., 1271 : 75-81, 2012). De fait, les effets néfastes sur la peau de ces agressions environnementales sont bien décrits. Ces agressions environnementales délétères peuvent perturber le développement de la peau, et en particulier de l'épiderme et de certaines de ses structures et fonctions, comme la fonction barrière, qui s'organisent après la naissance. Les conditions environnementales agressives entraînent ainsi des modifications de la structure et des fonctions cutanées qui se traduisent à terme par une certaine fragilité, réactivité, par des rides, perte de fermeté et de l'élasticité, sécheresse, brûlures, plaies superficielles ou plus importantes et autres effets indésirables cosmétiques.

Les inventeurs ont montré que l'exposition de la peau à des conditions environnementales agressives entraine une perte du potentiel des cellules souches. En particulier, les marqueurs définissant les cellules souches et, en particulier, les cellules souches basales, ne sont plus observés quand la peau est soumise à de telles agressions environnementales. Or les inventeurs ont montré que, de façon surprenante, les sucres en C7 tels que définis ci-dessus sont capables de protéger les cellules souches épidermiques de l'action délétère de ces agressions. En particulier, les inventeurs ont constaté que les sucres en C7 trouvés dans l'avocat, le perséitol et le mannoheptulose, ainsi que leurs dérivés résultant de l'estérification d'une ou plusieurs des fonctions hydroxyles du sucre avantageusement avec un acide gras, maintiennent l'expression des marqueurs définissant les cellules souches épidermiques dans les peaux, alors même que la peau subit des conditions environnementales agressives, ce qui démontre que lesdits sucres sont capables de protéger le potentiel des cellules souches épidermiques et, plus particulièrement, celui des cellules souches basales.

En revanche, ces mêmes sucres d'avocat n'affectent pas l'expression des marqueurs des cellules souches épidermiques dans des peaux qui n'ont pas été soumises à des agressions environnementales telles que les UV, ce qui suggère que lesdits sucres sont parfaitement tolérés par l'organisme non stressé. En fait, ils sont tellement bien tolérés qu'aucune différence d'expression desdits marqueurs n'a pu être détectée en l'absence d'agression environnementale. Ainsi, le profil d'expression des marqueurs des cellules souches que les inventeurs ont mis en évidence dans des peaux de différents âges est-il maintenu en présence des sucres d'avocat.

Ces composés sont donc parfaitement neutres pour la peau en absence d'agression, mais sont capables de restaurer la capacité des cellules souches dont, en particulier, les cellules souches basales, dans des peaux attaquées, comme par exemple des peaux irradiées. De façon plus générale, les sucres en C7 accompagnent le développement et l'organisation de la peau après la naissance en préservant les cellules souches. Ainsi, ces composés préservent-ils la capacité de la peau, et en particulier la peau des enfants à résister aux effets délétères des agressions environnementales.

A titre d'illustration, l'utilisation est une l'utilisation cosmétique d'une composition comprenant au moins un sucre en C7 ou dérivé de formule (I) suivante

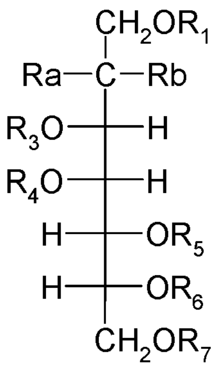

(I)

dans laquelle

Ra représente un atome d'hydrogène et Rb représente un $-OR_2$ ou CRaRb représente le radical CO ;

$R_1$, $R_2$, $R_3$, $R_4$, $R_5$, $R_6$ et $R_7$ représentent, indépendamment l'un de l'autre

- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique ;

et un excipient pharmaceutiquement acceptable pour la protection des cellules souches épidermiques et, en particulier, des cellules souches basales.

Selon un aspect ne faisant pas partie de l'invention, l'utilisation cosmétique de la composition a pour objet de prévenir les effets délétères pour la peau des agressions environnementales. Selon cet aspect, l'utilisation est une utilisation cosmétique d'une composition comprenant au moins un sucre en C7 ou dérivé de formule (I) et un excipient pharmaceutiquement acceptable pour la prévention des effets délétères pour la peau des agressions environnementales, caractérisée en ce que ladite composition protège les cellules souches épidermiques et, en particulier, les cellules souches basales.

Comme il est connu de l'homme du métier depuis longtemps, le *stratum corneum* joue un rôle indispensable au sein de la peau de par sa structure et son contact avec le milieu extérieur. Il constitue ainsi la principale barrière de la peau vis-à-vis des agressions environnementales, que celles-ci soient physiques (rayons UV...), chimiques (produits cosmétiques...), mécaniques (coupure, pincement...). Il est important à cet égard de remarquer que, pour que cette

fonction soit exercée correctement, il est indispensable que le *stratum corneum* soit présent dans toute son intégrité : sans cela, la perméabilité de la peau totale augmente. En particulier, il est important de maintenir le renouvellement cellulaire régulier de cette couche.

Plus particulièrement, l'utilisation est donc une utilisation cosmétique de la composition dans le but de préserver la fonction barrière. Selon cet aspect, l'utilisation est une utilisation cosmétique d'une composition comprenant au moins un sucre en C7 ou dérivé de formule (I) et un excipient pharmaceutiquement acceptable pour la préservation de la fonction barrière de la peau, caractérisée en ce que ladite composition protège les cellules souches épidermiques et, notamment, les cellules souches basales.

Il est aussi bien connu en particulier que les rayons UV représentent le principal facteur de vieillissement extrinsèque de la peau (voir par exemple Passeron et Ortonne, Presse Med 32 : 1474-1482, 2003), soit par interaction directe sur l'ADN cellulaire (mode d'action principal des UVB), soit de façon indirecte par le biais des formes actives de l'oxygène (mode d'action principal des UVA).

Au cours du processus de vieillissement, il apparaît différents signes caractéristiques sur la peau, qui se traduisent notamment par une modification de la structure et des fonctions cutanées avec par exemple une altération de la fonction barrière. Les signes de vieillissement existants, plus spécialement photo-induits, sont notamment les rides et les ridules dont l'apparition augmente avec l'âge et qui se traduisent par une dépression ou par des sillons à la surface de la peau. D'autres signes de vieillissement cutané, qui résultent d'un dysfonctionnement des principaux mécanismes biologiques intervenant au niveau de la peau, se traduisent par exemple par une desquamation, par une modification du teint, en l'occurrence un ternissement, et par une rugosité et une sécheresse en surface.

Selon l'invention, l'utilisation cosmétique de la composition a pour objet de prévenir le vieillissement de la peau. Ainsi, l'invention a pour objet l'utilisation cosmétique d'une composition comprenant au moins un sucre en C7 ou dérivé de formule (I) et un excipient pharmaceutiquement acceptable pour la prévention du vieillissement de la peau, caractérisée en ce que ladite composition protège les cellules souches épidermiques et, notamment, les cellules souches basales, et en ce qu'elle est destinée aux enfants.

Par ailleurs, les conditions environnementales difficiles donnent lieu souvent à l'apparition de plaies. Il est donc important que la peau soit capable de cicatriser correctement. Une "cicatrice" fait référence à la partie du tissu qui constitue la fermeture de plaies et, en particulier, des pertes de petites et/ou de grandes portions de tissus et d'organes. Le tissu cicatriciel en fait se forme à chaque interruption de la continuité de la peau (épiderme et/ou derme) causée par un événement pathologique ou traumatique dû aux agressions environnementales.

Les cellules souches épidermiques et, en particulier, les cellules souches basales, participent de l'homéostasie de la peau en général, et sont nécessaires au processus de cicatrisation. En effet, leur division permet de générer suffisamment de cellules différenciées pour constituer la cicatrice.

Selon un autre aspect ne faisant pas partie de l'invention, l'utilisation cosmétique de la composition a donc pour objet de favoriser la cicatrisation de la peau. Selon cet aspect, l'utilisation est une utilisation cosmétique d'une composition comprenant au moins un sucre en C7 ou dérivé de formule (I) et un excipient pharmaceutiquement acceptable pour favoriser la cicatrisation de la peau, caractérisée en ce que ladite composition protège les cellules souches épidermiques et, notamment, les cellules souches basales.

Il est aussi bien connu que les agressions environnementales sont à la source de nombreux cancers. Ainsi, les UV, à cause leurs propriétés génotoxiques, ont un rôle important dans l'induction et le développement des cancers de la peau. L'exposition aux rayons UV pourrait cibler les cellules souches de l'épiderme chez les enfants, ce qui augmenterait les risques de cancer de la peau à l'âge adulte (Volkmer et Greinert, Prog Biophys Mol Biol, 107(3) : 386-8, 2011). Il est donc important de préserver le potentiel des cellules souches et, en particulier, les cellules souches basales, pour prévenir les occurrences du cancer à un stade ultérieur de la vie.

Selon un autre aspect à titre d'illustration, la description a pour objet une composition comprenant au moins un sucre en C7 ou dérivé de formule (I) et un excipient pharmaceutiquement acceptable pour utilisation dans la prévention du cancer, de préférence le cancer de la peau, caractérisée en ce que ladite composition protège les cellules souches épidermiques et, notamment, les cellules souches basales.

En d'autres termes, selon cet aspect, il est ici décrit l'utilisation d'une composition comprenant au moins un sucre en C7 ou dérivé de formule (I) et un excipient pharmaceutiquement acceptable pour la fabrication d'un médicament pour prévenir le cancer, de préférence le cancer de la peau, caractérisée en ce que ladite composition protège les cellules souches épidermiques et, notamment, les cellules souches basales.

Il est bien entendu que les compositions cosmétiques ou thérapeutiques sont utilisées chez des mammifères, et de préférence des humains. Dans l'invention, lesdites compositions sont utilisées chez des enfants. Par « enfant », on entend ici un individu dont l'âge est inférieur à 16 ans. Sont ainsi compris dans la catégorie des enfants, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

Pour lever toute ambiguïté, le terme « enfant » utilisé ici sans autre précision doit être entendu dans son

acception la plus générale, c'est-à-dire comme se référant à une personne de moins de 16 ans. Un « adulte » est une personne qui n'est pas un enfant, autrement dit une personne âgée de plus de 16 ans.

Le D-mannoheptulose, premier cétoheptose identifié en 1916 par La Forge, de formule générale (II)

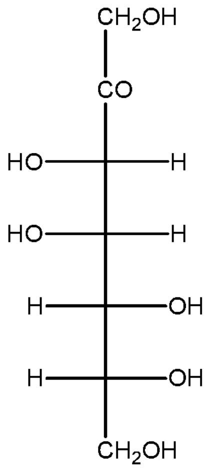

(II)

se retrouve dans certaines plantes, en particulier dans la luzerne (*Medicago sativa* L.), dans l'avocat, dans la figue (*Ficus officinalis* L.) dans l'orpin (*Sedum spectabile Bor.*) et dans la primevère (*Primula officinalis* Jacq.). Toutefois, c'est dans l'avocat, que l'on retrouve les teneurs les plus importantes en D-mannoheptulose. Le D-mannoheptulose a déjà été utilisé dans des applications thérapeutiques. Par exemple, la demande de brevet WO 95/03809 décrit l'utilisation du D-mannoheptulose, en tant qu'inhibiteur de glucokinase, pour inhiber le développement des cellules tumorales et la demande US 2003/0092669 décrit un complément alimentaire oral comprenant du D-mannoheptulose, qui permet de diminuer le taux d'insuline et qui permet ainsi une perte de poids.

Le perséitol, forme polyol du D-mannoheptulose, de formule générale (III)

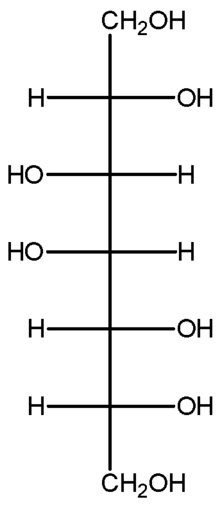

(III)

se retrouve également dans l'avocat, en particulier dans le fruit ou dans le noyau de l'avocat.

Il a été montré que le perséitol, associé à un ion potassium, permet d'inhiber l'incorporation de leucine-3H dans des cellules tumorales d'ascite sarcomateuse d'Ehrlich (Shibuya et al., Pure Appl. Chem., 71(6) : 1109-1113, 1999).

L'utilisation de ces sucres (perséitol et D-mannoheptulose) pour stimuler la synthèse des beta-défensines humaines (en particulier HBD-2) a déjà été décrite (WO 2005/115421). L'utilisation de ces sucres dans le traitement de candidoses et de pityrosporoses a également déjà été décrite (WO 2008/025847). Il avait enfin été montré que ces sucres pouvaient être utilisés pour traiter l'alopécie (WO 2011/073281) .

En revanche, il n'avait jamais été montré jusque-là que les sucres en C7 pouvaient protéger les cellules souches de l'épiderme et, en particulier, les cellules souches basales.

Par « cellule souche de l'épiderme » ou « cellule souche épidermique », on entend ici une cellule de l'épiderme

capable de renouvellement à long terme. Les cellules souches épidermiques comprennent, entre autres, les cellules souches folliculaires, les cellules souches sébacées et les cellules souches basales, ces dernières étant aussi appelées cellules souches épidermiques interfolliculaires. On entend par « cellules souches folliculaires », « cellules souches sébacées » et « cellules souches basales », les cellules souches situées respectivement dans la région du bulge/renflement du follicule pileux, dans les glandes sébacées et dans la couche basale de l'épiderme. Dans un mode préférentiel de réalisation de l'invention, les cellules souches épidermiques sont des cellules souches basales.

Plus précisément, on entend par cellule souche épidermique, une cellule dotée d'un potentiel élevé de renouvellement à long terme. Par « potentiel de renouvellement », on entend ici la capacité de subir au moins un cycle de division cellulaire. Un « potentiel élevé de renouvellement à long terme » représente donc la capacité d'une cellule d'entrer dans plusieurs cycles de division cellulaire successifs. Il est bien connu que les cellules différenciées de la peau ne sont pas capables d'effectuer plusieurs divisions successives (Fortunel et Martin, J Soc Biol, 202(1): 55-65, 2008). Il est entendu ici que «successif» ne signifie pas « consécutif » et qu'il peut exister des périodes pendant lesquelles une cellule souche reste quiescente sans toutefois perdre son potentiel élevé de renouvellement à long terme.

La conservation d'un potentiel élevé de renouvellement à long terme s'exprime par une division asymétrique produisant deux cellules différentes. La première cellule fille est une cellule souche identique à la cellule souche mère, tandis que la seconde est une cellule d'amplification transitoire qui se divise de façon limitée sur une courte période puis entre dans le processus de différenciation. Avantageusement, les cellules souches épidermiques sont donc en outre capables de générer au moins un type de cellule épidermique par différenciation. Autrement dit, la cellule d'amplification transitoire est capable de donner au moins un type de cellule épidermique par différentiation. Ladite cellule épidermique est par exemple un kératinocyte. La cellule d'amplification transitoire est par exemple capable de donner tous les types de cellules épidermiques par différentiation.

Comme on l'aura noté, la définition des cellules souches est avant tout fonctionnelle, puisqu'elle repose sur la propriété qu'ont ces cellules de garder la capacité de se diviser pendant une très longue période de temps. Cependant, il peut être difficile de vérifier si une cellule possède une telle propriété, en particulier quand ladite cellule est comprise dans une ou plusieurs couches de cellules : par exemple, dans un épiderme naturel (par exemple dans un organisme), dans un modèle de peau reconstituée, dans une culture monocouche ou bicouche, etc.

Il est donc avantageux de pouvoir identifier les cellules souches épidermiques sur la base d'autres critères. Plus précisément, il a été montré qu'il est particulièrement avantageux d'utiliser des marqueurs pour identifier les cellules souches.

Selon ce mode de réalisation préféré de l'invention, les sucres en C7 d'avocat et les compositions contenant ceux-ci permettent de maintenir l'expression d'un ou plusieurs marqueurs de cellules souches, notamment ceux des cellules souches basales.

Un « marqueur » est une substance ou une caractéristique biochimique, génétique, ou moléculaire distincte qui est spécifique des cellules souches épidermiques. Par exemple, il est connu que le colorant Hoechst 33342 (Sigma-Aldrich) est activement exporté par les cellules souches épidermiques (Larderet et al., Stem Cells 24(4) : 965-974, 2006).

Avantageusement, le « marqueur » est spécifiquement présent dans lesdites cellules souches épidermiques. Autrement dit, dans ce cas, le « marqueur » est exprimé de façon préférentielle dans les cellules souches épidermiques. Alternativement, le terme « marqueur » tel qu'on l'entend ici recouvre aussi les gènes et les protéines qui présentent une absence spécifique d'expression dans les cellules souches épidermiques.

L'expression d'un marqueur est maintenue par les sucres en C7 d'avocat et les compositions contenant ceux-ci si le niveau d'expression dudit marqueur n'est pas affecté par lesdits sucres ou composition. Autrement dit, les sucres en C7 et les compositions conduisent à un niveau d'expression substantiellement identique du marqueur qui est identique dans une cellule traitée avec lesdits sucres ou compositions et dans une cellule qui n'a pas été traitée.

Les sucres en C7 d'avocats et les compositions comprenant ceux-ci permettent par exemple de maintenir l'expression d'un marqueur d'une cellule souche épidermique, quel que soit l'âge du donneur de ladite cellule souche.

A titre illustratif, les sucres en C7 d'avocats et les compositions comprenant ceux-ci permettent de maintenir l'expression d'une cellule souche de l'épiderme subissant une agression environnementale quand le niveau d'expression d'un marqueur dans cette cellule souche épidermique attaquée et traitée avec lesdits sucres ou composition est substantiellement identique au niveau dudit marqueur dans une cellule souche épidermique témoin. Plus préférablement, selon cet aspect illustratif, ladite cellule souche épidermique témoin est une cellule souche épidermique qui n'a pas subi d'agression environnementale.

A titre illustratif, les sucres en C7 d'avocats et les compositions comprenant ceux-ci permettent de maintenir l'expression d'une cellule souche de l'épiderme irradiée aux UV quand le niveau d'expression d'un marqueur dans cette cellule souche épidermique irradiée et traitée avec lesdits sucres ou composition est substantiellement identique au niveau dudit marqueur dans une cellule souche épidermique témoin. Plus préférablement, selon cet aspect illustratif, ladite cellule souche épidermique témoin est une cellule souche épidermique qui n'a pas été irradiée.

Le marqueur est par exemple un marqueur génique, un marqueur protéique, un marqueur lipidique ou un marqueur métabolique. Pour chacun de ces types de marqueurs, de nombreuses méthodes sont à la disposition de

l'homme du métier pour mesurer l'expression dudit marqueur et ainsi identifier une différence d'expression entre les cellules souches épidermiques et les autres cellules de la peau.

Dans un premier mode de réalisation, ledit marqueur est un marqueur génique ou un marqueur protéique. Un marqueur génique ou protéique spécifique des cellules souches épidermiques est un gène ou une protéine, respectivement, exprimés de façon différentielle dans les cellules souches. Par exemple, un tel marqueur génique ou protéique peut être préférentiellement exprimé dans lesdites cellules souches ou bien, son expression peut être spécifiquement inhibée dans lesdites cellules souches.

De tels marqueurs ont été décrits dans de nombreuses publications (Larderet et al., Stem Cells 24(4) : 965-974, 2006 ; Cambiaso et al., Kératin, 13 : 9-15, 2007 ; Fortunel et Martin, J Soc Biol, 202(1) : 55-65, 2008 ; Craig et al., Mol Cancer, 9 : 195-207, 2010 ; Dereure, Ann Dermatol Venereol. 139(8-9) : 568-578, 2012 ; Dahl, J Cosmet Dermatol, 11: 297-306, 2012) et sont donc bien connus de l'homme du métier.

On citera par exemple les marqueurs ΔNp63, survivine, FN1 (fibronectin 1), MCSP (Melanoma-associated Chondroitin Sulfate Proteoglycan), LRIG1 (Leucine-rich repeats and immunoglobulin-like domains protein 1), GJA1 (connexin 43), NID1 (nidogen 1), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6) et ITGB4 (integrin beta 4) ou encore des marqueurs impliqués dans la signalisation et la régulation de l'activité des cellules souches comme Wnt/beta catenin, sonic hedgehog (SHH), NOTCH1 (Notch homolog 1, translocation-associated). Les marqueurs ΔNp63 et survivine sont des marqueurs de résistance à l'apoptose, ayant donc un rôle dans la survie des cellules souches. Les cytokératines 15 et 19 sont des marqueurs positifs des cellules souches, la cytokératine 15 étant un marqueur de survie de celles-ci. Le MCSP colocalise avec les intégrines dans les cellules qui ne se divisent pas, tandis que les intégrine beta1 (marqueur d'adhésion à la matrice extracellulaire de la membrane basale) et intégrine alpha 6 (constituant des hemidesmosomes marqueur de liaison des kératinocytes entre eux) sont des protéines de surface qui participent à la communication intercellulaire, régulent les processus de différenciation/prolifération, ainsi que l'interaction avec la niche. Le récepteur à la transférine CD71 est un marqueur de surface connu des cellules souches, qui est utilisé pour isoler, dans une population de cellules integrine-alpha6 positives, les cellules à haut pouvoir clonogéniques. Enfin, Lrig1 est un antagoniste du récepteur à l'Epidermal Growth Factor (EGFR), maintenant ainsi les cellules souches quiescentes, alors que le récepteur EGFR, qui est un marqueur dont l'absence caractérise les cellules souches, entraine au contraire les cellules dans la voie de la prolifération.

Cette liste n'est pas limitative et l'homme du métier saura identifier d'autres marqueurs de cellules souches de l'épiderme et de la niche en se servant de ses connaissances générales.

De façon préférentielle, le marqueur génique ou protéique selon l'invention est choisi parmi KRT15, NOTCH1, KRT19, ITGBP1 et ITGA6. De façon encore plus préférentielle, ledit marqueur génique ou protéique selon l'invention est ITGA6.

A titre illustratif, la composition comprenant un sucre en C7 sera donc capable de protéger les cellules souches si elle est capable de maintenir l'expression du marqueur génique ou protéique desdites cellules souches.

L'expression du marqueur génique ou protéique peut être détectée par tout moyen connu de l'homme du métier.

De façon générale, l'expression du marqueur génique ou protéique sera détectée in vitro à partir d'un échantillon cutané.

Par « échantillon cutané », on entend ici tout échantillon contenant des cellules de la peau. Les échantillons cutanés comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures d'épidermes et de peaux reconstruites et les cultures de muqueuses reconstruites. Comme il est souvent difficile de travailler sur des explants frais, il est particulièrement avantageux d'utiliser des cultures de cellules cutanées. Par exemple, les cellules cutanées comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du

tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage.

Dans ce cas, il peut exister une ou plusieurs étapes intermédiaires entre le prélèvement de l'échantillon de cellules cutanées et la mesure de l'expression du marqueur biologique, lesdites étapes correspondant à l'extraction à partir dudit échantillon de cellules cutanées d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc) ou de protéines à partir d'un échantillon cellulaire ne sont que des procédures de routine bien connues de l'homme du métier.

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en œuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus...). Ces technologies sont utilisées en routine par l'homme du métier et il n'est donc pas besoin de les détailler ici. Des exemples de mises en œuvre basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation

et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Selon une première variante avantageuse de l'invention, les sucres en C7 sont sous une forme libre (les fonctions hydroxyles ne sont pas estérifiées). La composition comprend donc un sucre en C7 choisi dans le groupe constitué par le mannoheptulose, le perséitol et leurs mélanges.

La source de D-mannoheptulose et/ou de perséitol peut être un extrait hydrosoluble de sucres d'avocat ou d'une autre plante. Autrement, le D-mannoheptulose et le perséitol sont disponibles commercialement (origine synthétique). Selon une variante avantageuse de l'invention, la source de D-mannoheptulose et/ou de perséitol est un extrait hydrosoluble de sucres d'avocat.

L'extrait hydrosoluble de sucres d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un un extrait hydrosoluble de sucres à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Redn,* plus avantageusement parmi les variétés *Hass, Fuerte et Reed.* De préférence, on retiendra les variétés *Hass, Fuerte, Ettinger et Bacon,* et plus avantageusement les variétés *Hass* et *Fuerte.*

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

Tableau 1

| | |
|---|---|
| Eau | **70-85** % |
| Protéines | **1,5-4,5** % |
| Lipides | **12-23** % |
| Sucres | **1,5-5** % |
| Fibres | **1,1-1,6** % |

De fait, l'avocat n'est pas particulièrement riche en saccharides. Cependant, la nature des monosaccharides solubles est tout à fait particulière, tels que le perséitol ou le D-mannoheptulose constitués de 7 atomes de carbone.

L'extrait hydrosoluble de sucres d'avocat est susceptible d'être obtenu par un procédé comprenant les étapes successives suivantes :

- obtention d'un tourteau d'avocat, avantageusement du fruit de l'avocat, par séchage de l'avocat puis extraction des lipides (huile) ; ensuite

- délipidation complète dudit tourteau, puis lavage à l'eau ou à un milieu hydroalcoolique puis décantation et centrifugation afin de récupérer une fraction soluble riche en sucres en C7 (élimination du gâteau) ;

- déminéralisation sur résine ionique de ladite fraction soluble, obtenue à l'étape précédente ; puis

- ultrafiltration à 10 000 daltons ; et

- le cas échéant, concentration sous vide et conditionnement.

La première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation, la zéodratation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en œuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue, ou encore par voie chimique, à l'aide d'un solvant tel que l'hexane, dans un extracteur de type soxhlet ou dans un extracteur continu à bande, de type De Smet®, notamment selon le procédé décrit dans la demande FR 2 843 027, ou par un procédé utilisant le $CO_2$ supercritique. Parmi les intérêts majeurs du procédé, l'huile co-produite constitue un produit bien évidemment directement valorisable. Le fruit sec et déshuilé, encore appelé tourteau, peut subir ensuite les étapes suivantes :

- délipidation complète, notamment à l'acétone et/ou à l'éthanol,
- décantation puis lavage du tourteau à l'eau et/ou un mélange hydroalcoolique,
- élimination du gâteau par centrifugation, filtration, récupération de la fraction soluble,
- concentration,
- déminéralisation par échange d'ions
- ultrafiltration avec un seuil de coupure de 10 kDa,
- concentration sous vide, ajout de conservateur et conditionnement.

De façon générale, l'extrait aqueux final peut contenir en poids 0,1 à 20 % de matière sèche, avantageusement 1 à 10 % de matière sèche, encore plus avantageusement 3 à 5 % de matière sèche. La teneur en sucres en C7, c'est-à-dire en D-mannoheptulose et en perséitol, dans la matière sèche est avantageusement comprise entre 50 et 100 %, plus particulièrement entre 65 et 90% en poids, par rapport au poids total de la matière sèche. Les données analytiques moyennes d'une solution aqueuse à 5 % d'extrait sec, obtenue par le procédé décrit précédemment, sont données dans le tableau 2 suivant :

Tableau 2

| | |
|---|---|
| pH (dilution ¼) | **3 - 5** |
| Sucres en C7 / matière sèche | **50 - 100** % |

La composition relative en sucres de l'extrait hydrosoluble, en poids par rapport au poids total de la matière sèche de l'extrait, répond avantageusement aux critères suivants (composition relative déterminée par HPLC ; high performance liquid chromatography = chromatographie liquide haute performance):

- D-mannoheptulose 0 à 100%, en particulier 5 à 80 %,
- Perséitol 0 à 100%, en particulier 5 à 80 %,
- Saccharose inférieur à 10%,
- Glucose inférieur à 10%,
- Fructose inférieur à 10%.

L'extrait hydrosoluble de sucre d'avocat comprend par exemple, par rapport au poids total de la matière sèche, 10 à 80% en poids de D-mannoheptulose plus avantageusement 15 à 70 % en poids de D-mannoheptulose. L'extrait hydrosoluble de sucres d'avocat comprend par exemple, par rapport au poids total de la matière sèche, 20 à 80% en poids de perséitol, plus avantageusement 25 à 70% en poids de perséitol.

A titre illustratif, la composition relative en sucres de l'extrait hydrosoluble, en poids par rapport au poids total de la matière sèche de l'extrait, répond aux critères suivants (composition relative déterminée par HPLC) :

- D-mannoheptulose 25 à 60 %,
- Perséitol 25 à 60 %,
- Saccharose inférieur à 10 %,

(suite)

| | |
|---|---|
| - Glucose | inférieur à 10 %, |
| - Fructose | inférieur à 10 %. |

D'une manière surprenante les inventeurs ont constaté un effet de synergie entre le D-mannoheptulose et/ou le perséitol et les sucres minoritaires (fructose, glucose, saccharose) présents dans l'extrait de sucres d'avocat.

L'extrait obtenu pourra éventuellement être séché selon les procédés connus de l'homme de l'art par lyophilisation ou atomisation par exemple, à l'aide ou non d'un support tel que par exemple les maltodextrines, dans le but d'obtenir une poudre solide (extrait sec), totalement hydrosoluble.

L'extrait obtenu pourra éventuellement être fractionné en chaque sucre purifié. Cette séparation et purification peut être réalisée par l'ensemble des techniques connues de l'homme de métier, parmi lesquelles on peut citer de façon non exhaustive la précipitation/filtration, la recristallisation, ou encore la séparation par chromatographie telle que le procédé I.S.M.B. (Improved Simulated Moving Bed).

Selon une deuxième variante avantageuse de l'invention, les sucres en C7, que sont avantageusement le D-mannoheptulose et le perséitol, sont au moins partiellement estérifiés avec un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique. En particulier les sucres en C7 sont au moins partiellement estérifiés avec un résidu d'acide gras. La chaine hydrocarbonée peut être linéaire ou ramifiée, elle est avantageusement linéaire.

Le radical R représente avantageusement un résidu d'un acide gras.

Les acides gras considérés sont plus particulièrement des acides gras à longue chaîne, c'est-à-dire pouvant posséder plus de 11 atomes de carbone et notamment plus de 14 atomes de carbone.

Leur chaine hydrocarbonée peut être saturée ou contenir une ou plusieurs doubles liaisons. À titre représentatif de ces acides gras, on peut notamment citer les acides gras saturés comme les acides palmitique ($C_{16}$), stéarique ($C_{18}$), arachidique ($C_{20}$), béhénique ($C_{22}$) et lignocérique ($C_{24}$) et les acides gras insaturés comme les acides palmitoléique ($C_{16}$), oléique ($C_{18}$), linoléique ($C_{18}$), linolénique notamment sous ses formes $\alpha$ et $\gamma$ (C18) et arachidonique ($C_{20}$).

En particulier le radical R est avantageusement choisi dans le groupe constitué par un radical stéaryle, linoléyle, oléyle, palmityle, lauryle, myristyle, arachidyle, béhényle, lauroléyle, myristoléyle, palmitoléyle, linolényle sous ses formes $\alpha$ et $\gamma$, et/ou arachidonyle.

Il est particulièrement avantageux de substituer la chaîne hydrocarbonée avec groupement $-SO_3M$ avec M représentant un atome d'hydrogène, un ion ammonium $NH_4^+$ ou un ion métallique (en particulier sodium).

Dans les dérivés de formule (I), les fonctions hydroxyles peuvent être substituées par le résidu du même acide gras ou par des résidus d'acides gras différents.

Les dérivés d'acides gras des sucres en C7 peuvent être obtenus par réaction d'estérification résultant de la mise en contact, dans les conditions appropriées, d'un ou plusieurs acides de formule HOOC-R (R ayant la même définition que dans les paragraphes précédents) avec le D-mannoheptulose et/ou le perséitol disponibles commercialement (origine synthétique) ou avec l'extrait hydrosoluble de sucres d'avocat décrit ci-dessus.

Selon une troisième variante avantageuse de l'invention, les sucres en C7, que sont avantageusement le D-mannoheptulose et le perséitol, sont au moins partiellement estérifiés avec un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique. La chaine hydrocarbonée peut être linéaire ou ramifiée, elle est avantageusement linéaire.

Le radical R représente avantageusement un résidu d'un acide à courte chaîne, c'est-à-dire pouvant posséder moins de 10 atomes de carbone et notamment moins de 8 atomes de carbone.

Leur chaîne hydrocarbonée peut être saturée ou contenir une ou plusieurs doubles liaisons. À titre représentatif de ces acides, on peut notamment citer l'acide acétique.

Il est particulièrement avantageux de substituer la chaîne hydrocarbonée avec groupement $-SO_3M$ avec M représentant un atome d'hydrogène, un ion ammonium $NH_4^+$ ou un ion métallique (en particulier sodium).

Dans les dérivés de formule (I), les fonctions hydroxyles peuvent être substituées par le résidu du même acide ou par des résidus d'acides différents.

Les dérivés d'acides des sucres en C7 peuvent être obtenus par réaction d'estérification résultant de la mise en contact, dans les conditions appropriées, d'un ou plusieurs acides de formule HOOC-R' (R' ayant la même définition que dans les paragraphes précédents) avec le D-mannoheptulose et/ou le perséitol disponibles commercialement (origine synthétique) ou avec l'extrait hydrosoluble de sucres d'avocat décrit ci-dessus.

Selon une quatrième variante avantageuse de l'invention, les sucres en C7, que sont avantageusement le D-mannoheptulose et le perséitol, sont au moins partiellement estérifiés avec un radical -(CO)-R et avec un radical -(CO)-R', R et R' ayant les mêmes définitions que celles données dans les deuxième et troisième variantes.

Les dérivés d'acides des sucres en C7 peuvent être obtenus par réaction d'estérification résultant de la mise en contact, dans les conditions appropriées, d'un ou plusieurs acides de formule HOOC-R et d'un ou plusieurs acides de formule HOOC-R' avec le D-mannoheptulose et/ou le perséitol disponibles commercialement (origine synthétique) ou avec l'extrait hydrosoluble de sucres d'avocat décrit ci-dessus.

On appelle les dérivés obtenus par les deuxième, troisième ou quatrième variantes dérivé d'acide de D-mannoheptulose ou respectivement de perséitol.

Dans l'une quelconque des deuxième, troisième ou quatrième variantes, le rapport entre le nombre de fonctions esters du composé de formule (I) et le nombre de fonctions hydroxyles initiales, ou taux d'estérification, pour une molécule de sucre, varie de 0,2 à 1. Par exemple, il est notamment inférieur ou égal à 0,6, et en particulier inférieur ou égal à 0,4.

Le degré d'estérification est contrôlé par la concentration des réactifs, la durée de réaction et la température de réaction. Il peut être mesuré par chromatographie, en particulier par chromatographie par exclusion stérique.

Selon l'une ou l'autre des quatre variantes, la composition comprend 0,001 à 30 % en poids de D-mannoheptulose ou de son dérivé d'acide, par rapport au poids total de ladite composition, et/ ou 0,001 à 30 % en poids de perséitol ou de son dérivé d'acide, par rapport au poids total de ladite composition. Par exemple, la composition comprend 0,002 à 5 % en poids de D-mannoheptulose ou de son dérivé d'acide, par rapport au poids total de ladite composition, et/ ou 0,002 à 5 % en poids de perséitol ou de son dérivé d'acide, par rapport au poids total de ladite composition.

L'extrait est par exemple utilisé en tant qu'agent actif dans une composition telle qu'une composition cosmétique, dermatologique ou pharmaceutique, qui peut comprendre un ou plusieurs excipients appropriés. La composition peut en outre comprendre au moins un autre composé actif en plus des sucres en C7. Cet autre composé peut être choisi parmi tous les composés et leurs équivalents fonctionnels, énoncés ci-dessous :

A titre illustratif, cet autre composé peut être en particulier choisi parmi des actifs classiquement utilisés en dermatologie ou cosmétique tels que les émollients, les actifs hydratants, les activateurs de la synthèse de kératine, les kératorégulateurs, les kératolytiques, les agents restructurant de la barrière cutanée, des agonistes PPARs (ou Peroxysome Proliferator Activated Receptor), les agonistes RXR ou LXR, les agents cicatrisants, les agents sébo-régulateurs, les agents anti-irritants, les agents apaisants, les agents anti-inflammatoires, les agents anti-oxydants et les agents anti-âge, les agents dépigmentants ou hypodépigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immuno-modulateurs.

A titre illustratif, les agents cicatrisants et/ou restructurants de la barrière cutanée pouvant être utilisés en association sont le panthénol (vitamine B5), l'arabinogalactane, l'oxyde de zinc, les céramides, le cholestérol, le squalane et les phospholipides.

A titre illustratif, les agents sébo-régulateurs pouvant être utilisés en association sont choisis dans le groupe constitué par les inhibiteurs de 5-alpha-réductase. Le zinc (et les dérivés de zinc tels que ses sels gluconate, salicylate et acide pyroglutamique) et la spironolactone, présentent aussi une activité sébo-suppresseur. D'autres séborégulateurs d'origine lipidique agissant sur la qualité du sébum, comme l'acide linoléique présentent également un intérêt.

A titre illustratif, l'agent anti-inflammatoire et/ou anti-irritant et/ou apaisant peut être l'arabinogalactane.

A titre illustratif, les actifs protecteurs solaires pouvant être utilisés en association sont des filtres et écrans solaires UVB et/ou UVA, tels les écrans ou filtres minéraux et/ou organiques connus de l'Homme du métier qui adaptera leur choix et leurs concentrations en fonction du degré de protection recherché.

Les conservateurs pouvant être utilisés en association sont par exemple ceux généralement utilisés en cosmétique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Cet autre composé peut être en particulier choisi parmi les extraits végétaux, en particulier :

- les huiles végétales telles que l'huile de soja et/ou l'huile de colza, l'huile d'avocat (WO2004/012496, WO2004/012752, WO2004/016106, WO2007/057439), l'huile de lupin et avantageusement l'huile de lupin blanc doux (WO98/47479), ou un mélange de ces huiles ;

- l'oléodistillat ou les concentrats d'huile végétale ou animale, notamment de tournesol, plus avantageusement des concentrats de tournesol linoléiques, tels que l'huile de tournesol concentrée en insaponifiables (Soline® - WO2001/21150), commercialisée par les Laboratoires Expanscience, les huiles concentrées en insaponifiables du type huile d'avocat, de colza, de maïs utiles notamment pour leur activité hydratante et/ou émolliente, cicatrisante et/ou restructurante de la barrière cutanée, anti-inflammatoire et/ou anti-irritante et/ou apaisante ;

- Les insaponifiables de végétaux ou d'huile végétale, avantageusement des furanes d'avocat (Avocadofurane®), pouvant être obtenus par le procédé décrit dans la demande internationale WO 01/21605 , les insaponifiables d'Avocat et/ou de Soja, plus particulièrement un mélange d'insaponifiables d'Avocat furaniques et d'insaponifiables de soja, avantageusement dans un rapport respectif d'environ 1/3 - 2/3 (tel que Piasclédine®), les insaponifiables de soja (tels qu'obtenus selon le procédé décrit dans le demande internationale WO 01/51596), les insaponifiables stéroliques (typiquement des insaponifiables dont la teneur en stérols, en méthylstérols et en alcool triterpéniques est comprise entre 20 et 95% en poids, de préférence 45-65% en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;
- Les peptides ou complexes d'acides aminés végétaux, en particulier les d'avocat (tel que ceux décrits dans la demande internationale WO2005/105123), les peptides de lupin (tel que ceux décrits dans la demande internationale WO 00/62789), l'extrait total de lupin (tel que ceux décrits dans la demande internationale WO2005/102259), les peptides de quinoa (tel que ceux décrits dans la demande internationale WO2008/080974), les peptides de maca (tel que ceux décrits dans la demande internationale WO2004/112742), les peptides de soja fermenté ou non, les peptides de riz (tel que ceux décrits dans la demande internationale WO2008/009709), utiles notamment pour leur activité hydratante et/ou émolliente (avocat), kératorégulatrice (lupin, quinoa), cicatrisante et/ou restructurante de la barrière (maca, quinoa, soja), anti-inflammatoire et/ou anti-irritante et/ou apaisante (lupin, quinoa), antioxydante (avocat), anti-âge (lupin, maca), pigmentante (riz), les peptides de schizandra (tel que ceux décrits dans la demande de brevet FR 0955344), l'extrait de graines d'*Acacia macrostachya* (tels que celui décrit dans la demande de brevet FR 0958525), l'extrait de graines de *Vigna unguiculata* (tels que celui décrit dans la demande de brevet FR 0958529) ; extrait de graines de passiflore (tels que celui décrit dans la demande de brevet FR 1262234) ;
- Le butyle avocadate (5 alpha Avocuta®), inhibiteur de la 5-alpha réductase (WO 01 /52837 et WO 02/06205) et typiquement, régulateur de la sécrétion séborrhée se trouvant augmentée dans l'acné et les pellicules ;
- Les extraits riches en polyphénols, et plus particulièrement les extraits de fruits d'avocat (tels que ceux décrits dans la demande FR 1 061 055), les extraits de feuilles de maca (tels que ceux décrits dans la demande FR 1 061 047), et les extraits de parties aériennes de *Gynandropsis gynandra* (tels que ceux décrits dans la demande FR 1 061 051),
- Le lupéol (FR 2 8 22 821, FR 2 857 596) utile notamment pour favoriser la cicatrisation ;
- Un beurre de Cupuaçu, particulièrement apprécié pour ses propriétés hydratantes.

Cet autre composé peut être en particulier choisi parmi les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline (de préférence la 2-undécyl-4,4-diméthyl-1,3-oxazoline, appelée OX-100 ou Cyclocéramide® ; WO2004050052, WO2004050079, et WO2004112741). Elles sont particulièrement utiles pour leur activité anti-inflammatoire et/ou anti-irritante et/ou apaisante, antioxydante, dépigmentant, immunomodulatrice.

Les sucres en C7 peuvent également être associés à des composés protecteurs ou activateurs des cellules souches comme Stemoxydine® (diethyl pyridine-2,4-dicarboxylate), Survicode™ (sodium cocoyl alaninate), Survixyl IS™ (pentapeptide-31),Defensil® (ctyldodecanol, Echium Planta-gineum Seed Oil, Cardiospermum Halicacabum Flower/Leaf/Vine Extract, Helianthus Annuus Sun-flower Seed Oil Unsaponifiables), Celligent® (Helianthus Annuus Sunflower Seed Oil, Ethyl Ferulate, Polyglyceryl-5 Trioleate, Rosmarinus Officinalis Leaf Extract, Aqua, Disodium Uridine Phosphate),Phycosaccharide AI®(alginic acid, sodium salt, hydrolysed), Phycojuvenine® (Laminaria digitata extract), PhytoCellTec™ à base d'extrait de cellules souches végétales de rose blanche des Alpes ou de raisin Gamay teinturier Fréaux ou de pomme Uttwiler spätlauber (Malus domestica) ou d'argan, cellules souches végétales extraites de vignes Vitis vinifera, cellules souches végétales de jeunes pousses de Christe Marine.

Toutes ces associations comprennent au moins un autre composé actif, en plus des sucres en C7, et peuvent comprendre deux, trois, quatre ou plus de composés actifs tels que décrits précédemment.

A titre illustratif, la composition peut être formulée sous la forme de différentes préparations adaptées à une administration topique, à une administration orale, rectale, vaginale, nasale, auriculaire ou bronchique, ainsi qu'à une administration parentérale.

Selon une première variante, les différentes préparations de l'invention sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Selon un aspect ne faisant pas partie de l'invention, la composition comprenant les sucres en C7 ayant les spécifications indiquées est particulièrement destinée à une utilisation cosmétique, pharmaceutique, dermatologique, nutraceutique.

Selon cet aspect, c'est à dire dans le cadre d'une utilisation cosmétique, pharmaceutique, ou dermatologique,

la composition sera avantageusement formulée sous la forme d'une préparation adaptée à une administration topique. La composition comprenant un extrait peptidique et osidique est particulièrement destinée à une utilisation cosmétique, pharmaceutique, ou dermatologique.

Dans le cadre d'une utilisation alimentaire, à visée nutritive ou cosmétique (« cosmet-food »), la composition sera avantageusement formulée sous la forme d'une préparation adaptée à une administration orale.

Il est également ici décrit l'utilisation des sucres en C7, pour la fabrication d'une composition cosmétique, pharmaceutique, dermatologique, d'une composition nutraceutique, ou d'un aliment fonctionnel.

Un aliment fonctionnel est un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

Il est ainsi ici décrit un aliment fonctionnel comprenant l'extrait selon l'invention.

Selon un aspect ne faisant pas partie de l'invention, il est ici décrit un extrait selon la description ou une composition selon la description pour son utilisation pour prévenir et/ou traiter :

- des troubles ou pathologies de la peau et/ou des muqueuses et/ou des phanères
- des troubles vasculaires
- des altérations du tissu adipeux.

En particulier, la composition ou l'extrait selon cet aspect est destiné à la prévention et/ou au traitement des réactions ou pathologies allergiques, inflammatoires, irritatives ou des troubles de la cicatrisation ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

Avantageusement, la composition ou l'extrait selon cet aspect peut être utilisé(e) pour la prévention et/ou le traitement des réactions, troubles ou pathologies :

- de la peau, telles que l'acné, la rosacée ou érythrocouperose, le psoriasis, les troubles vasculaires, la dermite du siège, la dermatite atopique, l'eczéma, la dermatite de contact, la dermatite irritative et en particulier la dermatite irritative du siège ou érythème fessier, la dermatite allergique, la dermite séborrhéique (croûte de lait), le psoriasis, la maladie de Lainer-Moussous, la peau sensible, la peau réactive, la peau sèche (xérose), la peau déshydratée,la peau endommagée par le soleil, par les radiations, par le vent, par le froid, par le chaud, par le stress, par la pollution, l'impétigo, la peau avec rougeur, l'érythème cutané, la peau âgée ou photoâgée, la peau photosensibilisée, la peau pigmentée (mélasma, pigmentation post-inflammatoire...), la peau dépigmentée (vitiligo), la peau avec cellulite, la peau relâchée, la peau avec vergetures, les dartres, les angiomes, les hémangiomes, les ichtyoses, les gerçures, les piqûres, les crevasses en particulier des seins, les brûlures, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes, verrues, prurigo strophulus, mycoses comme les candidoses et les pityrosporoses), radiologiques ou par déficit de l'immunité innée (peptides antimicrobiens) ou acquise (cellulaire, humorales, cytokines), et/ou

- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutisme, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

Il est également ici décrit un procédé de soin cosmétique de la peau et/ou des phanères et/ou des muqueuses, en vue d'améliorer leur état et/ou leur aspect, consistant à administrer une composition ou un extrait selon la présente description.

Il est ici décrit un procédé de soin cosmétique de la peau, en vue d'en prévenir le vieillissement, consistant à appliquer sur la peau une composition ou un extrait selon la présente description.

Selon un aspect ne faisant pas partie de l'invention, la composition ou l'extrait selon la présente description peut également être avantageusement utilisée dans la prévention et/ou le traitement des troubles de la cicatrisation.

Selon un aspect ne faisant pas partie de l'invention, la composition ou l'extrait selon la présente description peut également être avantageusement utilisée dans la prévention et/ou le traitement des troubles vasculaires, en particulier les rougeurs et les couperoses.

Selon un aspect ne faisant pas partie de l'invention, la composition ou l'extrait selon la présente description peut également être avantageusement utilisée dans la prévention et/ou le traitement des altérations du tissu adipeux. Les altérations des tissus adipeux sont en particulier la cellulite ou l'effet « peau d'orange ». La composition selon la description permet d'affermir la peau.

A titre illustratif, les modes d'administration, les posologies et les formes galéniques optimales des composés et compositions peuvent être déterminés selon les critères généralement pris en compte dans l'établissement d'un traitement pharmaceutique, en particulier dermatologique, cosmétique ou vétérinaire adapté à un patient ou à un animal, comme par exemple l'âge ou le poids corporel du patient ou de l'animal, la gravité de son état général, la tolérance au traitement, les effets secondaires constatés, le type de peau. A titre illustratif, en fonction du type d'administration souhaitée, la composition et/ou les composés actifs peuvent en outre comprendre au moins un excipient pharmaceutiquement acceptable, notamment dermatologiquement acceptable, ou un excipient cosmétiquement acceptable. Selon la première variante, on utilise par exemple un excipient adapté pour une administration par voie topique externe. A titre illustratif, la composition peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

**Légendes des figures**

Figure1 : Evolution des gènes des cellules souches en présence du perséose d'avocat.

Figure 2 : Immunomarquage de l'intégrine alpha 6, marqueur des cellules souches de la couche basale ; A : contrôle non irradié et non traité ; B : irradiation UVA+UVB ; C : irradiation UVA+UVB en présence de perséose d'avocat

Figure 3 : Immunomarquage de l'intégrine beta 1, marqueur des cellules souches de la couche basale ; A : contrôle non irradié et non traité ; B : irradiation UVA+UVB ; C : irradiation UVA+UVB en présence de perséose d'avocat

Les exemples qui suivent illustrent l'invention mais ne sont pas limitatifs.

**Exemples expérimentaux**

**1. Exemple 1 : préparation d'un extrait hydrosoluble de sucres d'avocat**

Les avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire. Ainsi sont obtenus de l'huile et un tourteau.

Le tourteau est alors broyé puis extrait, en présence d'éthanol à 70 % ou d'eau.

Les parties liquides et solides sont séparées par centrifugation par exemple. La fraction soluble (liquide) est reprise pour être purifiée et concentrée selon le mode opératoire suivant :

- *Déminéralisation à l'aide de résines échangeuses d'ions* : déminéralisation des heptuloses par passage sur résines $OH^-$, puis sur résine $H^+$.
- *Ultrafiltration sur 10 000 Da* : l'ultrafiltration est réalisée avec un système équipé de 4 membranes de seuil de coupure 10 kDa.
- *Concentration sous vide* : la concentration de l'extrait purifié est réalisée à l'aide d'un évaporateur sous vide jusqu'à l'obtention d'une matière sèche voisine de 4 %.
- *Conditionnement* : la concentration de l'extrait est ajustée à 5 % de matière sèche et on ajoute du conservateur, puis on filtre stérilement avec une membrane de 0,2 $\mu$m de seuil de coupure et on conditionne.

Le tableau 3 donne la composition de l'extrait de sucres d'avocat en C7, à 5 % de matière sèche, préparé suivant le procédé décrit ci-dessus :

Tableau 3

| Aspect | Solution de couleur jaune pâle |
|---|---|
| **Critères analytiques** | |
| Matière sèche | 5 % |
| pH (dilution ¼) | 7,0 |
| | |
| **Composition (%/matière sèche)** | |
| Saccharose | 3,0 |
| Glucose | 7,5 |
| D-mannoheptulose | 40,0 |
| Fructose | 8,6 |
| Perséitol | 40,0 |

Suivant ce même procédé, on a préparé deux autres extraits, dont la valeur du pH, l'absorbance et la teneur en sucres en C7 sont données dans le tableau 4. La teneur en sucres en C7 correspond à la somme du perséitol et du D-mannoheptulose analysée par HPLC.

Tableau 4

| Lot | 1 | 2 |
|---|---|---|
| Matière sèche | 5 % | 5% |
| pH (dilution ¼) | 5,9 | 5,4 |
| Sucres en C7 / matière sèche | 80,5 | 83,4 |

Les sucres d'avocat préparés par le procédé de l'exemple 1 et appelés « perséose d'avocat » ci-dessous ont été utilisés pour étudier l'expression des marqueurs de cellules souches dans différentes conditions.

**2. Effet du perséose d'avocat sur le profil d'expression des gènes, marqueurs, des cellules souches**

L'effet du perséose d'avocat sur l'expression des marqueurs de cellules souches a d'abord été testé dans un modèle de culture de kératinocytes.

2.1. Matériel et méthodes

2.1.1. Modèle biologique et traitement

Des échantillons de peau ont été prélevés (prélèvement préputial ou plastie mammaire, selon le sexe du donneur) sur des donneurs âgés de 1 mois, 3 mois, 3 ans, 6 ans et 11 ans, ainsi que sur des adultes. A partir de ces échantillons de peau, les kératinocytes épidermiques ont été extraits et cultivés en milieu SFM supplémenté, à 37°C et 5% $CO_2$.

Les différents kératinocytes ont été cultivés pendant 24 heures. Après incubation, le milieu a été remplacé par du milieu d'essai contenant ou non (témoin) le perséose d'avocat à 0,005%. Les cellules ont ensuite été incubées pendant 24 h. Toutes les conditions expérimentales ont été réalisées en triple. A la fin de l'incubation, les surnageants de culture ont été éliminés et les tapis cellulaires ont été immédiatement congelés à sec à -80°C.

2.1.2. Analyse de l'expression différentielle des gènes d'intérêt

L'expression des marqueurs a été évaluée par RT-qPCR sur les ARN messagers extraits des tapis cellulaires de chaque donneur. Les ARN totaux ont été extraits à partir de chaque échantillon à l'aide du réactif TriPure Isolation Reagent® (Roche) selon le protocole préconisé par le fabricant. Les ARN extraits ont été quantifiés à l'aide du spectrophotomètre NanoVue™ (GE Healthcare). Pour l'analyse par RT-PCR quantitative (RT-qPCR), les ARN préparés ont d'abord été rétro-transcrits en ADNc an présence d'oligo(dT) à l'aide de l'enzyme Superscript II. Les ADNc obtenus ont été quantifiés à l'aide du spectrophotomètre NanoVue™ (GE Healthcare) et leur concentration ajustée à 5 ng/μl.

Les réactions de PCR ont été réalisées par PCR quantitative avec les système « Light Cycler » (Roche Moleculear Systems Inc) et selon les procédures recommandées par le fournisseur.

Le mélange réactionnel (10 μl final) pour chaque échantillon contient 2,5 μl d'ADNc à 5 ng/μl, les amorces des différents marqueurs utilisés, le mélange réactionnel (Roche) contenant l'enzyme Taq DNA polymérase, le marqueur SYBR Green I et du $MgCl_2$.

L'incorporation de fluorescence dans l'ADN amplifié est mesurée en continu au cours des cycles de PCR. Ces mesures permettent d'obtenir des courbes d'intensité de la fluorescence en fonction des cycles de PCR et d'évaluer ainsi une valeur d'expression relative pour chaque marqueur.

Le nombre de cycles est déterminé à partir des points de sortie des courbes de fluorescence. Pour un même marqueur utilisé, plus un échantillon sort tard (nombre de cycles élevé), plus le nombre initial de copies de l'ARNm est faible.

La valeur d'expression relative (ER) est exprimée en unités arbitraires selon la formule suivante :

$$ER = (1/2^{\text{nombre de cycles}}) \times 10^6$$

Le gène de référence d'expression stable quelques soient les conditions expérimentales et qui a permis de normaliser les résultats est GAPDH.

**2.2. Résultats**

L'analyse génomique a permis de montrer que l'expression des gènes des cellules souches, KRT15, NOTCH1, KRT19, ITGBP1 et ITGA6, est importante après la naissance et diminue avec l'âge. Ce profil d'expression est maintenu en présence de perséose d'avocat (Figure 1), démontrant que le perséose d'avocat est bien toléré par les cellules.

**3. <u>Exemple comparatif : Évaluation de l'effet protecteur des cellules souches épidermiques du perséose d'avocat vis à vis d'une irradiation UVA + UVB</u>**

L'effet protecteur du perséose d'avocat sur les marqueurs des cellules souches a été testé avec un modèle de peau irradiée aux UV ou non.

**3.1. Matériel et méthodes**

3.1.1. Modèle biologique et traitement

Les explants de peaux sont mis à stabiliser 4 h à 37°C dans du milieu DMEM à 5 % de sérum.

Les explants sont déposés dans une plaque 24 puits contenant du milieu DMEM à 5 % de sérum. Les peaux sont prétraitées avec le perséose d'avocat formulé à 0,01% appliqués en topique au centre de l'explant. Du PBS est déposé sur les explants non traités considérés comme témoins.

Les peaux sont également prétraitées avec 0,005 % de perséose d'avocat dans le milieu de culture. Chaque condition est évaluée en double. Les peaux sont laissées pendant 24h à l'étuve. Les explants sont rincés au PBS puis transférés dans des plaques 6 puits pour être irradiés. La dose d'irradiation est de 10 $J/cm^2$ en UVA et de 200 $mJ/cm^2$ en UVB.

Les explants sont retraités comme précédemment puis remis 24 heures à l'incubateur.

3.1.2. Immunomarquage de protéines au sein du tissu

La technique d'inclusion permet de conserver la biopsie à l'état congelé sans fixation chimique (qui pourrait altérer les épitopes des antigènes d'intérêt). L'inclusion des peaux s'effectue en deux étapes : une étape de moulage de la peau dans une histocassette remplie d'OCT (Tissue-Tek), substance qui permet de conserver les biopsies de peaux sous forme congelée afin de réaliser des coupes au cryostat. Et une seconde étape dans l'azote liquide permettant de congeler et durcir la préparation. Ensuite des coupes d'épaisseur de 10μm sont réalisées au cryostat (enceinte réfrigérée pour réaliser au microtome des coupes congelées). Les coupes de peau sur lame de microscopie sont fixées dans de l'acétone pendant 10 min. Les sites non spécifiques sont saturés. Les anticorps primaires (anti-Δp63α, anti-α6 et anti-β1) sont laissés à incuber la nuit à 4°C. Puis le signal est révélé avec les anticorps secondaires couplés à un

fluorochrome 1 h à température ambiante dans le noir. En même temps que cette incubation, les noyaux cellulaires sont marqués au DAPI.

Les peaux sont observées en microscopie optique à fluorescence à l'aide du microscope Olympus CK 40. La visualisation de la fluorescence fait intervenir les filtres Rhodamine (TRITC, rouge), FITC (vert) et Dapi (bleu). Les peaux sont observées au grossissement X 40. Le logiciel permettant l'acquisition des images est le logiciel Archimed (Microvision).

L'acquisition des images a été réalisée avec un système d'imagerie à haute résolution automatisé (INCell Analyzer™1000, GE Healthcare). Pour chaque condition, 2 explants de peau ont été analysés et 5 saisies d'images numérisées ont été effectuées par explant, soit 10 images analysées.

### 3.2. Résultat

Les marquages ont été quantifiés par mesure de l'intensité de fluorescence de l'intégrine alpha6 rapportée au nombre de noyaux identifiés par le DAPI.

Comme indiqué dans le tableau 5, les cellules qui ont été irradiées perdent l'expression de l'intégrine alpha 6. En revanche, l'expression de ce marqueur est maintenue quand les cellules sont traitées avec le perséose d'avocat, indiquant que le perséose d'avocat permet de préserver le potentiel des cellules souches épidermiques

Tableau 5 : Quantification du marquage de l'intégrine alpha 6 dans la couche basale d'explants de peau

| | **INTEGRINE ALPHA 6** | **% par rapport au contrôle** |
|---|---|---|
| | (intensité de fluorescence) | |
| Contrôle non irradié et non traité | 49854 | 100% |
| Irradiation UVA+B | 10103 | 20% |
| Perséose d'avocat - Irradiation UVA+B | 40021 | 80% |

## 4. Exemples de formulations

### 4.1. Crème hydratante

Tableau 6

| **Matière première / Nom commercial** | % |
|---|---|
| CAPRYLO CAPRATE GLYC | 1 à 15% |
| HUILE DE TOURNESOLSR | 1 à 15% |
| ALCOOL CETYLIQUE PUR | 1 à 5% |
| GLYCERYL STEARATE CITRATE | 1 à 10% |
| CIRE ABEILLE | 1 à 5% |
| EUMULGINSG | 0 à 2% |
| ACETATE VITAMINE E | 0 à 1% |
| EAU PURIFIEE | QSP 100% |
| CARBOPOL ULTREZ20 | 0 à 1% |
| GLYCEROL | 1 à 10% |
| GOMME XANTHANE | 0 à 1% |
| LESSIVE SOUDEXI | 0 à 1% |
| CONSERVATEUR | 0 à 2% |
| **PERSEOSE AVOCAT** | **0 à 1%** |

**4.2. Lait restructurant**

Tableau 7

| Matière première / Nom commercial | % |
|---|---|
| EAU PURIFIEE | QSP 100% |
| HUILE DE TOURNESOLSR | 1 à 10% |
| HUILE DE COPRAH HYDRO | 1 à 10% |
| HUILE D'AMANDE DOUCE | 1 à 10% |
| HUILE DE MAIS | 1 à 10% |
| MONOSTEARATE GLYCEROL | 1 à 10% |
| ACIDE STEARIQUE | 1 à 10% |
| CONSERVATEUR | 0 à 2% |
| ALCOOL CETYLIQUE C16-C18 | 0 à 2% |
| ACETATE VITAMINE E | 0 à 1% |
| LESSIVE SOUDEXI | 0 à 1% |
| **PERSEOSE AVOCAT** | **0 à 1%** |

**4.3. Eau nettoyante**

Tableau 8

| Matière première / Nom commercial | % |
|---|---|
| EAU PURIFIEE | QSP 100% |
| GLYCEROL | 1 à 10% |
| SODIUM COCOYL GLUTAMATE | 1 à 10% |
| CONSERVATEUR | 0 à 2% |
| RICIN HYDROBUTETH26 | 0 à 2% |
| ALLANTOINE | 0 à 2% |
| ACIDE TARTRIQUE | 0 à 2% |
| ALOE VERA POUDRESR | 0 à 2% |
| EXTRAIT SAPONAIRESR | 0 à 2% |
| LESSIVE SOUDEXI | 0 à 2% |
| **PERSEOSE AVOCAT** | **0 à 1%** |

**4.4. Shampooing**

Tableau 9

| Matière première / Nom commercial | de |
|---|---|
| EAU PURIFIEE | QSP 100% |

(suite)

| **Matière première / Nom commercial** | **de** |
|---|---|
| COCAMIDO PROPYL BETAINE | 1 à 10% |
| GLYCEROL | 1 à 10% |
| COCOGLUCOSIDEXI | 1 à 10% |
| SODIUM MYRETH SULFATE | 1 à 10% |
| CONSERVATEUR | 0 à 2% |
| DISTEARATEPEG6000 | 0 à 2% |
| POLYQUARTERNIUMJR400XI | 0 à 2% |
| PANTHENOL DEXTROGYRE | 0 à 2% |
| ACIDE CITRIQUE HYDXI | 0 à 2% |
| **PERSEOSE AVOCAT** | **0 à 1%** |

**4.5. Crème solaire SPF 50+**

Tableau 10

| **Matière première / Nom commercial** | **de** |
|---|---|
| CAPRYLATE/CAPRATEDECOPRAH | 5 à 20% |
| DICAPRYLYLCARBONATE | 5 à 20% |
| CAP RYLOCAPRATEGLYC | 5 à 20% |
| MIGLYOLGELB | 5 à 20% |
| TITANIDIOXIDJOJOBAESTERS | 1 à 15 % |
| TINOSORBS | 1 à 5 % |
| DIETAMIN HYDBENZO YHEXBENZ | 1 à 10 % |
| ETHYL HEXYL TRIAZONE | 1 à 10 % |
| HUILE D'AVOCAT HAREF | 1 à 5% |
| ALPHA TOCOPHEROL | 0.05 à 1% |
| LAURYL GLUCOSE-GLYSTEARATXI | 2 à 12 % |
| EAU PURIFIEE | QSP 100% |
| GLYCEROL | 1 à 10% |
| GOMME XANTHANE | 0 à 1% |
| CONSERVATEUR | 0 à 2% |
| POTASSIUM CETYL PHOSPHATE | 0 à 2% |
| LESSIVE SOUDEXI | 0 à 2% |
| PHENYL BENZIMIDAZO SULFONAC | 1 à 5% |
| **PERSEOSE AVOCAT** | **0 à 1%** |

**Revendications**

**1.** Utilisation cosmétique d'une composition comprenant au moins un sucre en C7 ou dérivé de formule (I) suivante

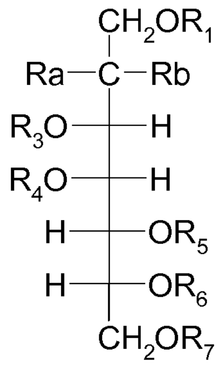

(I)

dans laquelle
Ra représente un atome d'hydrogène et Rb représente un $-OR_2$ ou CRaRb représente le radical CO ;
$R_1$, $R_2$, $R_3$, $R_4$, $R_5$, $R_6$ et $R_7$ représentent, indépendamment l'un de l'autre

- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique ;

et un excipient pharmaceutiquement acceptable
pour la prévention du vieillissement de la peau,
**caractérisée en ce que** ladite composition protège les cellules souches épidermiques, notamment les cellules souches basales, et **en ce qu'**elle est destinée aux enfants.

2. Utilisation selon la revendication 1, **caractérisée en ce que** ladite composition permet de maintenir l'expression d'un ou plusieurs marqueurs de cellules souches.

3. Utilisation selon la revendication 2, **caractérisée en ce que** ledit marqueur est un marqueur génique ou protéique.

4. Utilisation selon l'une quelconque des revendications 2 ou 3, **caractérisée en ce que** ledit marqueur est choisi parmi ΔNp63, FN1 (fibronectin 1), MCSP (Melanoma-associated Chondroitin Sulfate Proteoglycan), LRIG1 (Leucine-rich repeats and immunoglobulin-like domains protein 1), GJA1 (connexin 43), NID1 (nidogen 1), NOTCH1 (Notch homolog 1, translocation-associated), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6) et ITGB4 (integrin beta 4).

5. Utilisation selon l'une quelconque des revendications 2 à 4, **caractérisée en ce que** ledit marqueur est ITGA6 (integrin alpha 6).

6. Utilisation selon l'une quelconque des revendications 1 à 5, **caractérisée en ce que** le degré d'estérification, pour une molécule de sucre, est compris entre 0,2 et 1.

7. Utilisation selon l'une quelconque des revendications 1 à 6, **caractérisée en ce que** le radical R représente un résidu d'un acide gras, en particulier un radical stéaryle, linoléyle, oléyle, palmityle, lauryle, myristyle, arachidyle, béhényle, lauroléyle, myristoléyle, palmitoléyle, linolényle sous ses formes $\alpha$ et $\gamma$, et/ou arachidonyle.

8. Utilisation selon l'une quelconque des revendications 1 à 7, **caractérisée en ce que** la composition comprend un sucre en C7 choisi dans le groupe constitué par le mannoheptulose, le perséitol et leurs mélanges.

9. Utilisation selon la revendication 8, **caractérisée en ce que** la composition comprend 0,001 à 30 % en poids de D-mannoheptulose ou de son dérivé d'acide, par rapport au poids total de ladite composition, et/ ou 0,001 à 30 % en poids de perséitol ou de son dérivé d'acide, par rapport au poids total de ladite composition.

10. Utilisation selon l'une quelconque des revendications 8 ou 9, **caractérisée en ce que** la source de D-mannoheptulose et/ou de perséitol est un extrait hydrosoluble de sucres d'avocat, avantageusement susceptible d'être obtenu par un procédé comprenant les étapes successives suivantes :

- obtention d'un tourteau d'avocat, avantageusement du fruit de l'avocat, par séchage de l'avocat puis extraction des lipides ; ensuite
- cryobroyage et délipidation complète dudit tourteau, puis décantation et centrifugation afin de récupérer une fraction soluble riche en sucres en C7 (élimination du gâteau) ;
- déminéralisation sur résine ionique de ladite fraction soluble, obtenue à l'étape précédente ; puis
- ultrafiltration à 10 000 daltons ; et
- concentration sous vide et conditionnement.

11. Utilisation selon la revendication 10, **caractérisée en ce que** l'extrait hydrosoluble de sucres d'avocat comprend en poids, par rapport au poids total de la matière sèche de l'extrait (composition relative déterminée par HPLC):

| | |
|---|---|
| - D-mannoheptulose | 5 à 80 % |
| - Perséitol | 5 à 80% |
| - Saccharose | inférieur à 10% |
| - Glucose | inférieur à 10% |
| - Fructose | inférieur à 10% |

12. Utilisation selon l'une quelconque des revendications 1 à 11, **caractérisée en ce que** ladite composition comprend en outre un actif choisi dans le groupe constitué par :

- les huiles végétales, de préférence l'huile de soja, l'huile de colza, l'huile d'avocat, l'huile de lupin, et avantageusement l'huile de lupin blanc doux, ou un mélange de ces huiles ;
- les oléodistillats ou les concentrats d'huile végétale ou animale, de préférence de tournesol, d'avocat, de colza, de maïs et de palme et avantageusement concentrés en insaponifiables ;
- les insaponifiables de végétaux ou d'huile végétale, de préférence les insaponifiables d'avocats, les insaponifiables de soja ou leurs mélanges, avantageusement des furanes d'avocat, et en particulier, un mélange d'insaponifiables d'Avocat furaniques et d'insaponifiables de Soja dans un rapport respectif d'environ 1/3-2/3, les insaponifiables stéroliques, les phytostérols, les esters de stérols et les dérivés vitaminiques ;
- les peptides ou complexes d'acides aminés végétaux, de préférence les peptides d'avocat, les peptides de lupin, l'extrait total de lupin, les peptides de quinoa, les peptides de Maca, les peptides de soja fermentés ou non, les peptides de riz, un extrait de graines *d'Acacia macrostachya,* un extrait de graines de *Vigna unguiculata,* un extrait de graines de passiflore ;
- le butyle avocadate ;
- les extraits riches en polyphénols, de préférence les extraits de fruits d'avocat, les extraits de parties aériennes de Gynandropsis gynandra et les extraits de feuilles de Maca,
- le lupéol,
- un beurre de Cupuaçu ;
- les oxazolines, de préférence la 2-undécyl-4-hydroxyméthyl-4-méthyl-l,3-oxazoline, la 2-undécyl-4,4-diméthyl-l,3-oxazoline, la (E)-4,4-diméthyl-2-heptadéc-8-ényl-l,3-oxazoline, la 4-hydroxyméthyl-4-méthyl-2- heptadécyl-l,3-oxazoline, la (E)-4-hydroxyméthyl-4-méthyl-2-heptadéc-8- ényl-1,3-oxazoline, la 2-undécyl-4-éthyl-4-hydroxyméthyl-1,3-oxazoline et la 2-undécyl-4,4-diméthyl-l,3-oxazoline, et
- leurs mélanges.

13. Utilisation selon l'une quelconque des revendications 1 à 12, **caractérisée en ce que** ladite composition comprend en outre un actif choisi parmi les composés protecteurs ou activateurs des cellules souches, notamment le diethyl pyridine-2,4-dicarboxylate, le sodium cocoyl alaninate, le pentapeptide-31,le ctyldodecanol, Echium Planta-gineum Seed Oil, Cardiospermum Halicacabum Flower/Leaf/Vine Extract, Helianthus Annuus Sun-flower Seed Oil Unsaponifiables, le Helianthus Annuus Sunflower Seed Oil, Ethyl Ferulate, Polyglyceryl-5 Trioleate, Rosmarinus Officinalis

Leaf Extract, Aqua, Disodium Uridine Phosphate, l'acide alginique hydrolysé, l'extrait de Laminaria digitata, l'extrait de cellules souches végétales de rose blanche des Alpes ou de raisin Gamay teinturier Fréaux ou de pomme Uttwiler spätlauber (Malus domestica) ou d'argan, les cellules souches végétales extraites de vignes Vitis vinifera, et les cellules souches végétales de jeunes pousses de Christe Marine.

**14.** Utilisation selon l'une quelconque des revendications 1 à 13, **caractérisée en ce que** ladite composition est destinée à une administration topique ou par voie orale.

## Patentansprüche

**1.** Kosmetische Verwendung einer Zusammensetzung, die mindestens einen C7-Zucker oder Derivat der folgenden Formel (I) umfasst

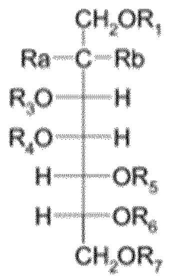
(I)

wobei
Ra ein Wasserstoffatom darstellt und Rb ein $-OR_2$ darstellt oder CRaRb das Radikal CO darstellt;
$R_1$, $R_2$, $R_3$, $R_4$, $R_5$, $R_6$ und $R_7$ unabhängig voneinander darstellen

- ein Wasserstoffatom oder
- ein Radikal -(CO)-R, wobei R eine gesättigte oder ungesättigte Kohlenwasserstoffkette darstellt, die 11 bis 24 Kohlenstoffatome enthält, eventuell substituiert durch einen oder mehrere Substituenten, die aus der Gruppe ausgewählt sind, die von den Hydroxyradikalen (-OH), den Ethoxyradikalen ($-OC_2H_5$) und der Gruppe $-SO_3M$ gebildet ist mit M, das ein Wasserstoffatom, ein Ammoniumion $NH_4^+$ oder ein Metallion darstellt; oder
- ein Radikal -(CO)-R', wobei R' eine gesättigte oder ungesättigte Kohlenwasserstoffkette darstellt, die 2 bis 10 Kohlenstoffatome enthält, eventuell substituiert durch einen oder mehrere Substituenten, die aus der Gruppe ausgewählt sind, die von den Hydroxyradikalen (-OH), den Ethoxyradikalen ($-OC_2H_5$) und der Gruppe $-SO_3M$ gebildet ist mit M, das ein Wasserstoffatom, ein Ammoniumion $NH_4^+$ oder ein Metallion darstellt;

und einen pharmazeutisch akzeptablen Hilfsstoff zur Vorbeugung der Hautalterung,
**dadurch gekennzeichnet, dass** die Zusammensetzung die epidermalen Stammzellen, insbesondere die basalen Stammzellen, schützt, und dass sie für Kinder bestimmt ist.

**2.** Verwendung nach Anspruch 1, **dadurch gekennzeichnet, dass** die Zusammensetzung erlaubt, die Expression von einem oder mehreren Stammzellenmarkern aufrechtzuerhalten.

**3.** Verwendung nach Anspruch 2, **dadurch gekennzeichnet, dass** der Marker ein genetischer oder Proteinmarker ist.

**4.** Verwendung nach einem der Ansprüche 2 oder 3, **dadurch gekennzeichnet, dass** der Marker aus ΔNρ63, FN1 (Fibronectin 1), MCSP (Melanoma- associated Chondroitin Sulfate Proteoglycan), LRIG1 (Leucine-rich repeats and immunoglobulin-like domains protein 1), GJA1 (Connexin 43), NID1 (Nidogen 1), NOTCH1 (Notch homolog 1, translocation-associated), KRT15 (Keratin 15), KRT19 (Keratin 19), EGFR (Epidermal growth factor receptor), CD71 (Transferrin receptor), DSG3 (Desmoglein 3), ITGB1BP1 (Integrin betal binding protein), ITGA6 (Integrin alpha 6) und ITGB4 (Integrin beta 4) ausgewählt ist.

**5.** Verwendung nach einem der Ansprüche 2 bis 4, **dadurch gekennzeichnet, dass** der Marker ITGA6 (Integrin alpha 6) ist.

6. Verwendung nach einem der Ansprüche 1 bis 5, **dadurch gekennzeichnet, dass** der Veresterungsgrad für ein Zuckermolekül zwischen 0,2 und 1 liegt.

7. Verwendung nach einem der Ansprüche 1 bis 6, **dadurch gekennzeichnet, dass** das Radikal R einen Rest einer Fettsäure darstellt, vor allem ein Stearyl-, Linoleyl-, Oleyl-, Palmityl-, Lauryl-, Myristyl-, Arachidyl-, Behenyl-, Lauroleyl-, Myristoleyl-, Palmitoleyl-, Linolenylradikal in seinen Formen $\alpha$ und $\gamma$, und/oder Arachidonylradikal.

8. Verwendung nach einem der Ansprüche 1 bis 7, **dadurch gekennzeichnet, dass** die Zusammensetzung einen C7-Zucker umfasst, der aus der Gruppe ausgewählt ist, die von der Mannoheptulose, dem Perseitol und deren Gemischen ausgewählt ist.

9. Verwendung nach Anspruch 8, **dadurch gekennzeichnet, dass** die Zusammensetzung 0,001 bis 30 Gew.-% D-Mannoheptulose oder ihres Säurederivats in Bezug auf das Gesamtgewicht der Zusammensetzung und/oder 0,001 bis 30 Gew.-% Perseitol oder seines Säurederivats in Bezug auf das Gesamtgewicht der Zusammensetzung umfasst.

10. Verwendung nach einem der Ansprüche 8 oder 9, **dadurch gekennzeichnet, dass** die Quelle von D-Mannoheptulose und/oder von Perseitol ein wasserlöslicher Extrakt aus Avocadozuckern ist, der in vorteilhafter Weise durch ein Verfahren gewinnbar ist, das die folgenden aufeinanderfolgenden Schritte aufweist:

    - Erhalten eines Avocadokuchens, in vorteilhafter Weise von der Avocadofrucht, durch Trocknen der Avocado, dann Extraktion der Lipide; danach
    - Kaltzerkleinern und vollständige Delipidierung des Kuchens, dann Abscheiden und Zentrifugieren, um eine lösliche Fraktion, die reich an C7-Zuckern ist, zurückzugewinnen (Entfernen des Kuchens);
    - Entmineralisieren der in vorangegangenem Schritt erhaltenen löslichen Fraktion auf ionischem Harz; dann
    - Ultrafiltration bei 10.000 Dalton; und
    - Vakuumkonzentration und Verpackung.

11. Verwendung nach Anspruch 10, **dadurch gekennzeichnet, dass** der wasserlösliche Avocadozuckerextrakt in Gewicht in Bezug auf das Gesamtgewicht der Trockenmasse des Extrakts umfasst (relative Zusammensetzung, bestimmt durch HPLC):

| | |
|---|---|
| - D-Mannoheptulose | 5 bis 80 % |
| - Perseitol | 5 bis 80 % |
| - Saccharose | unter 10 % |
| - Glucose | unter 10 % |
| - Fructose | unter 10 % |

12. Verwendung nach einem der Ansprüche 1 bis 11, **dadurch gekennzeichnet, dass** die Zusammensetzung ferner einen Wirkstoff umfasst, der aus der Gruppe ausgewählt ist, die gebildet ist von:

    - den pflanzlichen Ölen, vor allem dem Sojaöl, dem Rapsöl, dem Avocadoöl, dem Lupinenöl und in vorteilhafter Weise dem Öl der weißen Süßlupine oder einem Gemisch dieser Öle;
    - den Öldestillaten oder den Konzentraten aus pflanzlichem oder tierischen Öl, vor allem aus Sonnenblume, Avocado, Raps, Mais und Palme und in vorteilhafter Weise konzentriert an Unverseifbaren;
    - den Unverseifbaren von Pflanzen oder Pflanzenöl, vor allem den Avocado-Unverseifbaren, den Soja-Unverseifbaren oder deren Gemischen, in vorteilhafter Weise den Avocadofuranen, und insbesondere einem Gemisch aus furanischen Avocado--Unverseifbaren und Soja-Unverseifbaren in einem Verhältnis von zirka 1/3-2/3, den sterolischen Unverseifbaren, den Phytosterolen, den Sterolestern und den Vitaminderivaten;
    - den Peptiden oder Komplexen pflanzlicher Aminosäuren, vor allem den Avocadopeptiden, den Lupinenpeptiden, dem Lupinen-Totalextrakt, den Quinoapeptiden, den Macapeptiden, den fermentierten oder nicht fermentierten Sojapeptiden, den Reispeptiden, einem Extrakt aus Saat von *Acacia macrostachya,* einem Extrakt aus Saat von *Vigna unguiculata,* einem Extrakt aus Saat von Passionsblume;
    - dem Avocadatbutyl;
    - den an Polyphenolen reichen Extrakten, vor allem den Avocadofruchtextrakten, den Extrakten der oberirdischen Teile von Gynandropsis gynandra und den Extrakten von Macablättern,
    - dem Lupeol,

- einer Cupuaçu-Butter;
- den Oxazolinen, vor allem 2-Undecyl-4-hydroxymethyl-4-methyl-1,3-oxazolin, 2-Undecyl-4,4-dimethyl-1,3-oxazolin, (E)-4,4-Dimethyl-2-heptadec-8-enyl-1,3-oxazolin, 4-Hydroxymethyl-4-methyl-2-heptadecyl-1,3-oxazolin, (E)-4-Hydroxymethyl-4-methyl-2-heptadec-8-enyl-1,3-oxazolin, 2-Undecyl-4-ethyl-4-hydroxymethyl-1,3-oxazolin und 2-Undecyl-4,4-dimethyl-1,3-oxazolin, und
- deren Gemischen.

**13.** Verwendung nach einem der Ansprüche 1 bis 12, **dadurch gekennzeichnet, dass** die Zusammensetzung ferner einen Wirkstoff umfasst, der aus den Verbindungen ausgewählt ist, die Stammzellen schützen oder aktivieren, insbesondere Diethylpyridin-2,4-dicarboxylat, Natriumcocoylalaninat, Pentapeptid-31, Ctyldodecanol, Echium Planta-gineum Seed Oil, Cardiospermum Halicacabum Flower/Leaf/Vine Extract, Helianthus Annuus Sun-flower Seed Oil Unverseifbaren, Helianthus Annuus Sunflower Seed Oil, Ethyl Ferulate, Polyglyceryl-5 Trioleate, Rosmarinus Officinalis Leaf Extract, Aqua, Disodium Uridine Phosphate, hydrolysierter Alginsäure, dem Extrakt von Laminaria digitata, dem Extrakt pflanzlicher Stammzellen der weißen Alpenrose oder der Traube Gamay Teinturier Fréaux oder des Apfels Uttwiler spätlauber (Malus domestica) oder Argan, den pflanzlichen, aus Wein Vitis vinifera extrahierten Stammzellen und den pflanzlichen Stammzellen von jungen Meerfencheltrieben.

**14.** Verwendung nach einem der Ansprüche 1 bis 13, **dadurch gekennzeichnet, dass** die Zusammensetzung für eine topische oder orale Verabreichung bestimmt ist.

## Claims

**1.** A cosmetic use of a composition comprising at least one C7 sugar or derivative of the following formula (I)

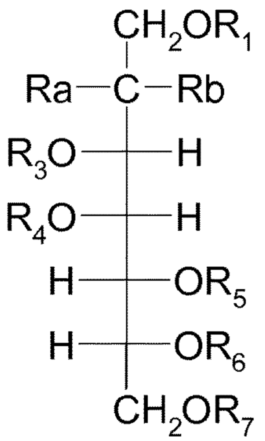

(I)

wherein
Ra represents a hydrogen atom and Rb represents an $-OR_2$ group or CRaRb represents the CO radical;
$R_1$, $R_2$, $R_3$, $R_4$, $R_5$, $R_6$ and $R_7$ represent, independently of each other

- a hydrogen atom or
- a -(CO)-R radical wherein R represents a saturated or unsaturated hydrocarbon chain containing 11 to 24 carbon atoms, optionally substituted by one or more substituents selected from the group consisting of hydroxy radicals (-OH), ethoxy radicals ($-OC_2H_5$) and - $SO_3M$ group with M representing a hydrogen atom, an ammonium ion $NH_4^+$ or a metal ion; or
- a -(CO)-R' radical wherein R' represents a saturated or unsaturated hydrocarbon chain containing 2 to 10 carbon atoms, optionally substituted by one or more substituents selected from the group consisting of hydroxy radicals (-OH), ethoxy radicals ($-OC_2H_5$) and - $SO_3M$ group with M representing a hydrogen atom, an ammonium ion $NH_4^+$ or a metal ion;

and a pharmaceutically acceptable excipient for preventing skin aging,
**characterised in that** said composition protects the epidermal stem cells, in particular the basal stem cells, and **in that** it is intended for children.

2. The use according to claim 1, **characterised in that** said composition allows to maintain the expression of one or more markers of stem cells.

3. The use according to claim 2, **characterised in that** said marker is a gene or protein marker.

4. The use according to any one of claims 2 or 3, **characterised in that** said marker is selected from ΔNp63, FN1 (fibronectin 1), MCSP (Melanoma-associated Chondroitin Sulfate Proteolyglycan), LRIG1 (Leucine-rich repeats and immunoglobulin-like domains protein 1), GJA1 (connexin 43), NIDI (nidogen 1), NOTCH1 (Notch homolog 1, translocation-associated), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6) and ITGB4 (integrin beta 4).

5. The use according to any one of claims 2 to 4, **characterised in that** said marker is ITGA6 (integrin alpha 6).

6. The use according to any one of claims 1 to 5, **characterised in that** the degree of esterification, for a sugar molecule, is comprised between 0.2 and 1.

7. The use according to any one of claims 1 to 6, **characterised in that** the radical R represents a residue of a fatty acid, in particular a stearyl, linoleyl, oleyl, palmityl, lauryl, myristyl, arachidyl, behenyl, lauroleyl, myristoleyl, palmitoleyl, linolenyl in its $\alpha$ and $\gamma$ forms, and/or arachidonyl radical.

8. The use according to any one of claims 1 to 7, **characterised in that** the composition comprises a C7 sugar selected from the group consisting of mannoheptulose, perseitol and mixtures thereof.

9. The use according to claim 8, **characterised in that** the composition comprises 0.001 to 30% by weight of D-mannoheptulose or the acid derivative thereof, relative to the total weight of said composition, and/or 0.001 to 30% by weight of perseitol or the acid derivative thereof, relative to the total weight of said composition.

10. The use according to any one of claims 8 or 9, **characterised in that** the source of D-mannoheptulose and/or perseitol is an avocado sugar water-soluble extract, advantageously obtainable by a method comprising the following successive steps:

- obtaining an avocado cake, advantageously from the fruit of the avocado, by drying of the avocado then extraction of the lipids; then
- cryogrinding and complete delipidation of said cake, then decantation and centrifugation in order to recover a soluble fraction rich in C7 sugars (elimination of the cake);
- demineralisation on ionic resin of said soluble fraction, obtained in the preceding step; then
- ultrafiltration at 10,000 daltons; and
- concentration under vacuum and packaging.

11. The use according to claim 10, **characterised in that** the avocado sugar water-soluble extract comprises by weight, relative to the total weight of the dry matter of the extract (relative composition determined by HPLC) :

| | |
|---|---|
| - D-mannoheptulose | 5 to 80% |
| - Perseitol | 5 to 80% |
| - Sucrose | less than 10% |
| - Glucose | less than 10% |
| - Fructose | less than 10% |

12. The use according to any one of claims 1 to 11, **characterised in that** said composition further comprises an active agent selected from the group consisting of:

- plant oils, preferably soy oil, rapeseed oil, avocado oil, lupin oil, and advantageously sweet white lupin oil, or a mixture of these oils;
- oleodistillates or concentrates of plant or animal oil, preferably sunflower, avocado, rapeseed, corn and palm oil and advantageously concentrated in unsaponifiables;

- unsaponifiables of plants or plant oil, preferably avocado unsaponifiables, soy unsaponifiables or mixtures thereof, advantageously avocado furans, and in particular, a mixture of furanic unsaponifiables of avocado and unsaponifiables of soy in a respective ratio of about 1/3-2/3, sterolic unsaponifiables, phytosterols, sterol esters and vitamin derivatives;
- plant peptides or complexes of amino acids, preferably avocado peptides, lupin peptides, total lupin extract, quinoa peptides, Maca peptides, fermented or non-fermented soy peptides, rice peptides, an extract of *Acacia macrostachya* seeds, an extract of *Vigna unguiculata* seeds, an extract of passionflower seeds;
- butyl avocadate;
- polyphenol-rich extracts, preferably extracts of avocado fruits, extracts of aerial parts of Gynandropsis gynandra and extracts of Maca leaves,
- lupeol,
- a Cupuacu butter;
- oxazolines, preferably 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-methyl-2-heptadecyl-1,3-oxazoline, (E)-4-hydroxymethyl-4-methyl-2-heptadec-8-enyl-1,3-oxazoline, 2-undecyl-4-ethyl-4-hydroxymethyl-1,3-oxazoline and 2-undecyl-4,4-dimethyl-1,3-oxazoline, and
- mixtures thereof.

**13.** The use according to any one of claims 1 to 12, **characterised in that** said composition further comprises an active agent selected from the compounds which protect or activate stem cells, in particular diethyl pyridine-2,4-dicarboxylate, sodium cocoyl alaninate, pentapeptide-31, ctyldodecanol, Echium Planta-gineum Seed Oil, Cardiospermum Halicacabum Flower/Leaf/Vine Extract, Helianthus Annuus Sun-flower Seed Oil Unsaponifiables, Helianthus Annuus Sunflower Seed Oil, Ethyl Ferulate, Polyglyceryl-5 Trioleate, Rosmarinus Officinalis Leaf Extract, Aqua, Disodium Uridine Phosphate, hydrolysed alginic acid, the Laminaria digitata extract, the plant stem cell extract of white alpine rose or Gamay Teinturier Freaux grape or Uttwiler spätlauber apple (Malus domestica) or argan, plant stem cells extracted from Vitis vinifera vines, and plant stem cells from young shoots of Christe Marine.

**14.** The use according to any one of claims 1 to 13, **characterised in that** said composition is intended for topical or oral administration.

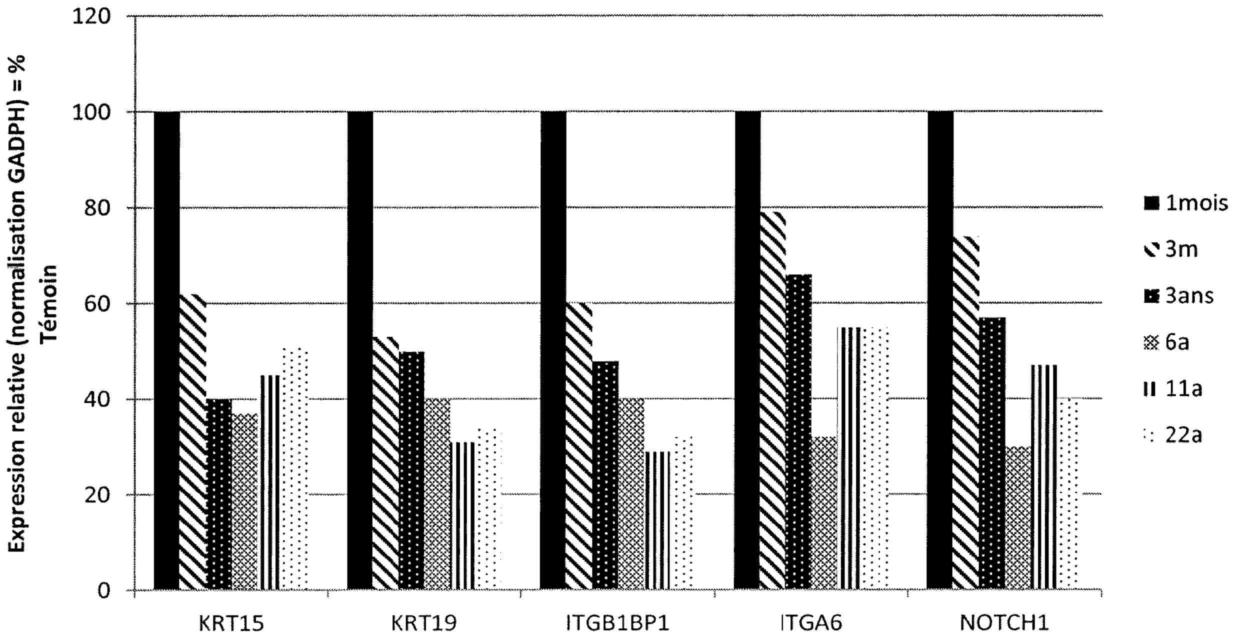
Expression relative (normalisation GADPH) = % Témoin
120
100
80
60
40
20
0
KRT15
KRT19
ITGB1BP1
ITGA6
NOTCH1
1mois
3m
3ans
6a
11a
22a

Figure 1

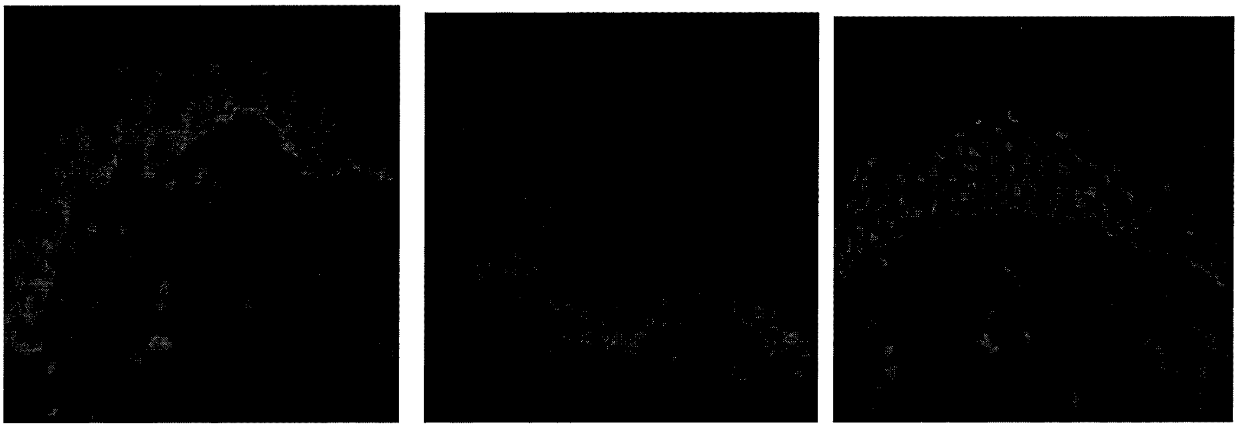

A B C

Figure 2

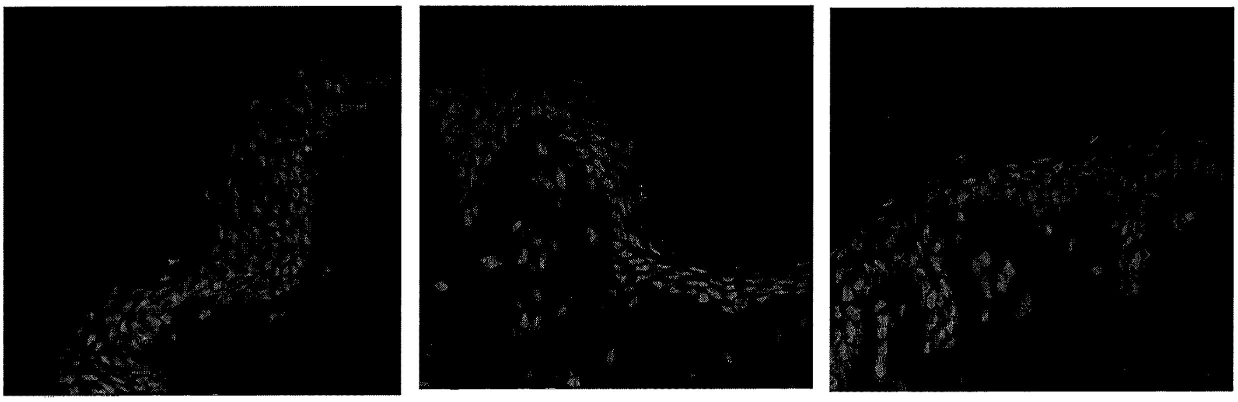

A B C

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9503809 A
- US 20030092669 A
- WO 2005115421 A
- WO 2008025847 A
- WO 2011073281 A
- US 4882127 A
- US 4849077 A
- US 7556922 B
- US 6723513 B
- WO 03066896 A
- WO 2007111924 A
- US 20080020392 A
- WO 2006084132 A
- US 20090186349 A
- US 20090181860 A
- US 20090181385 A
- US 20060275782 A
- EP 1141399 B1
- FR 2843027
- WO 2004012496 A
- WO 2004012752 A
- WO 2004016106 A
- WO 2007057439 A
- WO 9847479 A
- WO 200121150 A
- WO 0121605 A
- WO 0151596 A
- WO 2005105123 A
- WO 0062789 A
- WO 2005102259 A
- WO 2008080974 A
- WO 2004112742 A
- WO 2008009709 A
- FR 0955344
- FR 0958525
- FR 0958529
- FR 1262234
- WO 0152837 A
- WO 0206205 A
- FR 1061055
- FR 1061047
- FR 1061051
- FR 2822821
- FR 2857596
- WO 2004050052 A
- WO 2004050079 A
- WO 2004112741 A


### Littérature non-brevet citée dans la description


- **CHIOU et al.** *Skin Pharmacol Physiol,* 2004, vol. 17, 57-66
- **NIKOLOVSKI et al.** *J Invest Dermatol,* 2008, vol. 128, 1728-1736
- **STAMATAS et al.** *Pediatr Dermatol,* 2010, vol. 27, 125-131
- **FLUHR et al.** *Br J Dermatol,* 2012, vol. 166 (3), 483-90
- **DAHL.** *J Cosmet Dermatol,* 2012, vol. 11, 297-306
- **ECKERT et al.** *Biochim Biophys Acta,* 2013, vol. 1830, 2427-2434
- **FUCHS.** *J Cell Biol,* 2008, vol. 182 (2), 273-284
- **DEREURE.** *Ann Dermatol Venereol.,* 2012, vol. 139 (8-9), 568-578
- **VALACCHI et al.** *Ann. N.Y. Acad. Sci.,* 2012, vol. 1271, 75-81
- **PASSERON ; ORTONNE.** *Presse Med,* 2003, vol. 32, 1474-1482
- **VOLKMER ; GREINERT.** *Prog Biophys Mol Biol,* 2011, vol. 107 (3), 386-8
- **SHIBUYA et al.** *Pure Appl. Chem.,* 1999, vol. 71 (6), 1109-1113
- **FORTUNEL ; MARTIN.** *J Soc Biol,* 2008, vol. 202 (1), 55-65
- **LARDERET et al.** *Stem Cells,* 2006, vol. 24 (4), 965-974
- **CAMBIASO et al.** *Kératin,* 2007, vol. 13, 9-15
- **CRAIG et al.** *Mol Cancer,* 2010, vol. 9, 195-207
- **SHENDURE ; JI.** *Nat Biotechnol.,* 2008, vol. 26 (10), 1135-45
- **PIHLAK et al.** *Nat Biotechnol.,* 2008, vol. 26 (6), 676-684
- **FULLER et al.** *Nature Biotechnol.,* 2009, vol. 27 (11), 1013-1023
- **MARDIS.** *Genome Med.,* 2009, vol. 1 (4), 40
- **METZKER.** *Nature Rev. Genet.,* 2010, vol. 11 (1), 31-46